# EUROPEAN PATENT APPLICATION

(11) **EP 4 212 176 A1**
(43) Date of publication of application: **19.07.2023**
(21) Application number: 22210104.0
(22) Date of filing: 19.06.2017
(51) Int. Cl.: A61K 39/395, A61K 31/7088, A61K 31/7105, A61K 45/00, A61K 48/00, A61P 9/00, A61P 21/00, A61K 47/68, C07K 16/28

(54) **ANTIBODY-DRUG CONJUGATE**

(30) Priority: 20.06.2016 JP 2016122187
(62) Divisional of application: 17815351.6
(71) Applicant: GenAhead Bio, Inc., Fujisawa-shi Kanagawa 251-0012 (JP)
(72) Inventor: Sugo, Tsukasa, Fujisawa-shi, Kanagawa, 251-0012 (JP); Miyata, Kenichi, Fujisawa-shi, Kanagawa, 251-0012 (JP); Ohtaki, Tetsuya, Tsukuba-shi, Ibaraki, 305-0031 (JP)
(74) Representative: Hoffmann Eitle

(57) **Abstract**

[Problem to be solved]

The present invention provides an antibody-drug conjugate (ADC). The present invention also provides a conjugate of an anti-CD71 antibody or an antigen-binding fragment thereof with a drug. The present invention further provides a conjugate of Fab' with a drug. The present invention further provides a composition comprising the conjugate for delivering the drug to a tissue or a cell of muscle or the like.

[Solution]

A conjugate of an anti-CD71 antibody or an antigen-binding fragment thereof with a drug, and a conjugate of Fab' of an antibody with a drug.

## Description

### Technical Field

The present invention relates to an antibody-drug conjugate (ADC), in particular to a conjugate of an antigen-binding fragment (e.g., Fab') of an anti-CD71 antibody with a drug. The present invention provides a composition comprising the conjugate for delivering a drug to muscle.

### [Background Art]

CD71 is a transferrin receptor, and serves to incorporate iron into a cell by binding to holo-transferrin bound to iron and transporting the holo-transferrin into the cell by receptor-mediated endocytosis. It is believed that CD71 is recycled to the cell membrane after incorporated from the cell membrane into the cell by endocytosis.

Since transferrin is incorporated into a cell by CD71, studies have been conducted to incorporate the conjugated drug into the CD71-expressing cell by conjugating the transferrin with a drug (Non-Patent Literatures 1 to 4). It has been reported that the conjugate of transferrin with DNA itself cannot be incorporated into a cell, but when the polyethyleneimine is complexed with the conjugate, the DNA can be incorporated into the cell (Non-Patent Literature 5).

Muscle is considered as a tissue into which drugs are hardly delivered. This is considered because the vascular endothelium in muscle is configured to pave vessels on pavement, and thus there are no holes for allowing passage of substances. Since such a vascular endothelial configuration or the like act as a barrier, it is difficult to efficiently deliver a drug into muscle (Non-Patent Literatures 6 and 7).

Non-Patent Literature 8 discloses monomeric antibody-siRNA conjugates against Her2, TENB2 or the like. Non-Patent Literature 9 discloses bispecific digoxigenin-binding antibodies in which siRNA is linked to an antibody against Her2, IGF1-R, CD22 or the like via digoxigenin.

Non-Patent Literature 10 discloses that siRNA is delivered to muscle by a Fab' fragment of anti-CD71 antibody, but this literature was presented after the priority date of the present application by the inventors and discloses a part of content of the basic application of the present application.

Patent Literature 1 discloses that the presence or absence of anti-tumor effect is determined by the amino acid sequence of anti-CD71 antibody, and further discloses amino acid sequences of an antibody having an anti-tumor effect.

### Citations List

### Patent Literature

Patent Literature 1: WO2007/000671

### Non-Patent Literatures

Non-Patent Literature 1: E. Wagner et al., PNAS, 87: 3410-3414, 1990
Non-Patent Literature 2: E. Chang et al., Hum Gene Ther., 8: 467-475, 1997
Non-Patent Literature 3: M. Davis et al., Bioconjug. Chem., 14: 1122-1132, 2003
Non-Patent Literature 4: Y. Sato et al., FASEB J., 14: 2018-2118, 2000
Non-Patent Literature 5: K.W. Liang et al., Molecular Therapy, 1(3): 236-243, 2000
Non-Patent Literature 6: B. Rippe et al., Physiol. Rev. 74: 163-219, 1994
Non-Patent Literature 7: J.A. Wolff et al., Adv. Genet. 54: 1-20, 2005
Non-Patent Literature 8: T.L. Cuellar et al., Nucleic Acid Research, 43(2): 1189-1203, 2015
Non-Patent Literature 9: B. Schneider et al., Molecular Therapy-Nucleic Acids, 1, e46, 2012
Non-Patent Literature 10: T. Sugo et al., Journal of Controlled Release, 237: 1-13, 2016

### Summary of Invention

### Technical Problem

An object of the present invention is to provide a method for effectively delivering a drug such as a nucleic acid to a tissue or a cell of muscle or the like.

### Solution to Problem

The present inventors have found that a conjugate of an anti-CD71 antibody or an antigen-binding fragment thereof with a drug is excellently delivered to muscle. Further, the present inventors have found that the drug delivered to muscle may exert desired activities (for example, when the conjugate of an anti-CD71 antibody or an antigen-binding fragment thereof with siRNA against myostatin is delivered to muscle, an effect on increasing quantity of muscle can be exhibited) in the muscle. The present invention has been made based on these findings.

That is, the present invention provides the following inventions.
[1] A conjugate of an anti-CD71 antibody or an antigen-binding fragment thereof with a drug (which may be sometimes referred to as the conjugate of the present invention, herein.).
[2] The conjugate according to [1] above, wherein the antigen-binding fragment is Fab'.
[3] The conjugate according to [1] or [2] above, wherein the anti-CD71 antibody or the antigen-binding fragment thereof is Fab' of the anti-CD71 antibody.
[4] The conjugate according to any one of [1] to [3] above, wherein the drug is a nucleic acid.
[5] The conjugate according to any one of [1] to [4] above, wherein the drug is RNA.
[6] The conjugate according to any one of [1] to [5] above, wherein the drug is mRNA.
[7] The conjugate according to any one of [1] to [5] above, wherein the drug is siRNA.
[8] The conjugate according to any one of [1] to [4] above, wherein the drug is an antisense oligonucleotide.
[9] The conjugate according to any one of [1] to [8] above, wherein the anti-CD71 antibody or the antigen-binding fragment thereof is linked to the drug by a linker.
[10] A composition comprising the conjugate according to any one of [1] to [9] above, for use in delivering a drug to at least one selected from cardiac muscle and skeletal muscle.
[11] Use of an anti-CD71 antibody or an antigen-binding fragment thereof, for delivering a drug to at least one selected from cardiac muscle and skeletal muscle.
[12] An anti-CD71 antibody or antigen-binding fragment thereof, for use in delivering a drug to at least one selected from cardiac muscle and skeletal muscle.
[13] A medicament comprising the conjugate according to any one of [1] to [9] above.
[14] The medicament according to [13] above, wherein the medicament is a preventive and/or therapeutic agent for a disease of muscle.
[15] A method for prevention and/or therapy for a disease of muscle, comprising administering to a mammal an effective amount of the conjugate according to any one of [1] to [9] above.
[16] Use of the conjugate according to any one of [1] to [9] above for producing a preventive and/or therapeutic agent for a disease of muscle.
[17] The conjugate according to any one of [1] to [9] above for use in prevention and/or therapy for a disease of muscle.
[18] A method for producing a conjugate of Fab' of an anti-CD71 antibody with a drug, comprising
   reacting a thiol group of the Fab' with a drug having a functional group reactive with the thiol group, or
   reacting a thiol group of the Fab' with a drug bound to a linker having a functional group reactive with the thiol group.

### Advantageous Effects of Invention

According to the present invention, a drug may be effectively delivered to a cell or a tissue of muscle or the like such as skeletal muscle and cardiac muscle. Further, according to the present invention, the drug may prevent or therapeutically treat a disease of the cell or the tissue of muscle or the like by being effectively delivered to a cell or a tissue of muscle or the like such as skeletal muscle and cardiac muscle.

### Brief Description of Drawings

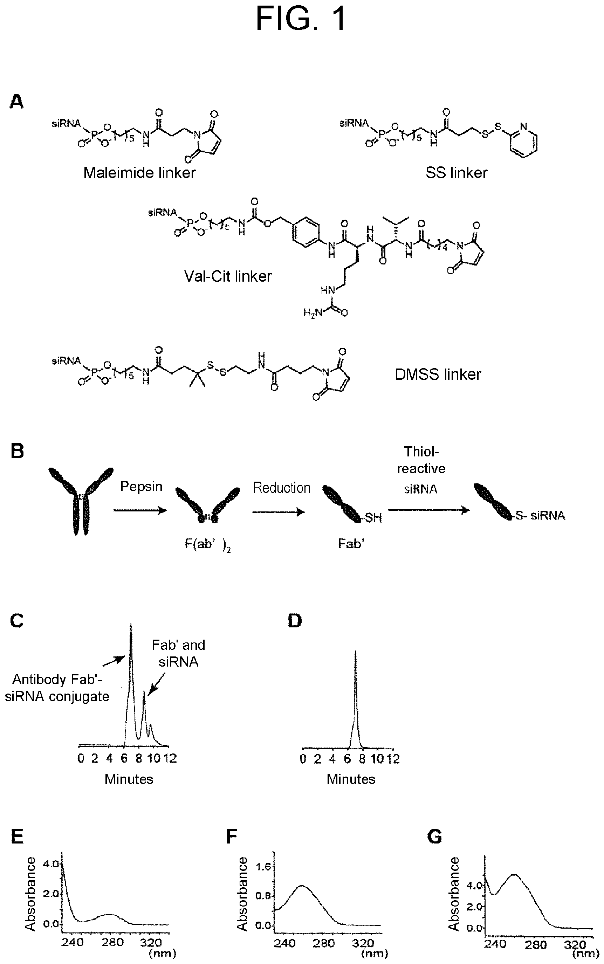

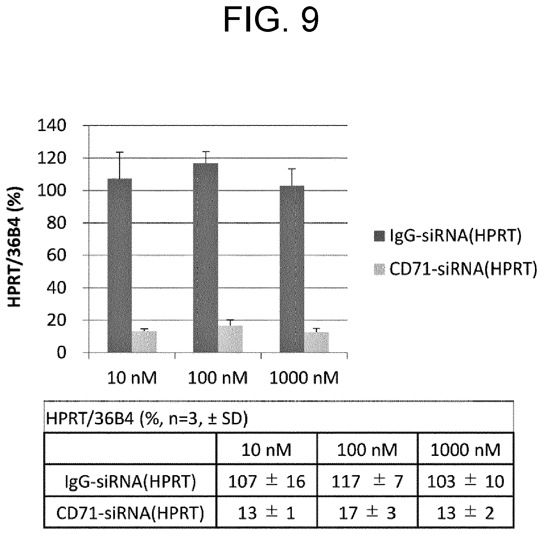

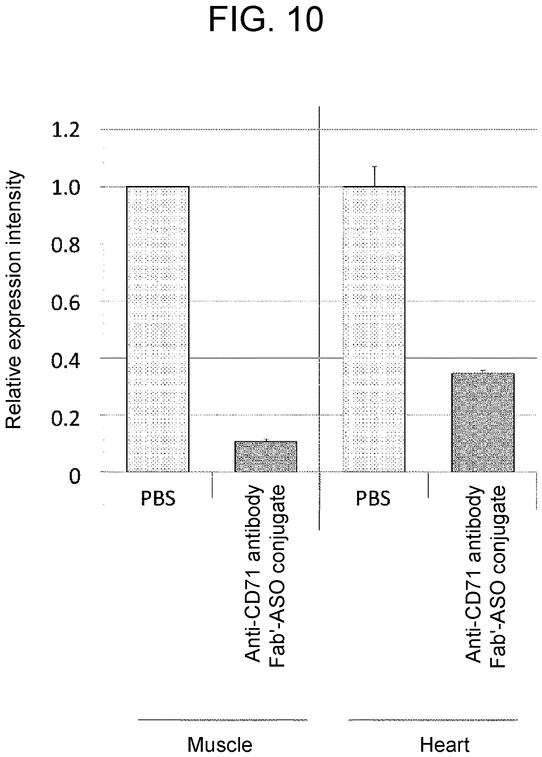

[Figure 1] Figure 1 shows a preparation example of a conjugate in which the antibody is Fab' and the drug is siRNA, as an example of an antibody-drug conjugate.
Figure 1A shows an example of introducing a thiol-reactive group to the siRNA. Figure 1B shows a scheme of obtaining a conjugate by digesting an antibody and reducing to Fab', and reacting the thiol group of Fab' to the thiol-reactive group of siRNA. Figure 1C shows a chromatogram obtained by size exclusion chromatography-HPLC. Figure 1D is a chromatogram which shows peaks of the conjugate, indicating that the conjugate can be obtained in high purity. Figure 1E shows the absorption spectrum of Fab'. Figure 1F shows the absorption spectrum of siRNA. Figure 1G shows the absorption spectrum of the conjugate.

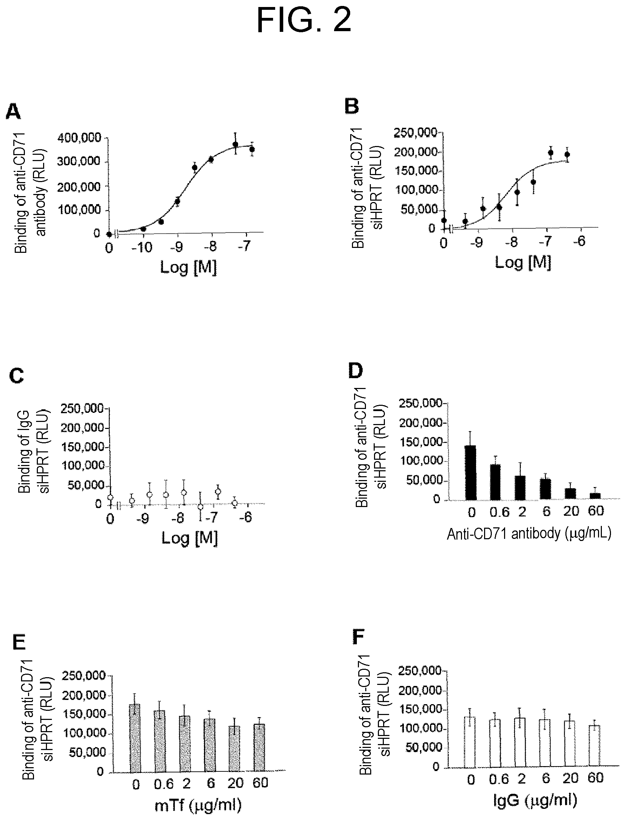

[Figure 2] Figure 2 shows an evaluation result of binding ability of an antibody-drug conjugate in which the antibody is Fab' of an anti-CD71 antibody purchased from Bio X Cell (West Lebanon, NH) (clone R17 217.1.3) (herein also referred to as "clone R17") and the drug is siRNA, as an example of an antibody-drug conjugate. Figure 2A shows the binding activity of clone R17 to CD71 fixed on the plate surface. Figure 2B shows the binding activity of the conjugate of Fab' of clone R17 with siRNA (specifically, siRNA against HPRT) to CD71. Figure 2C shows that a conjugate of Fab' of isotype control IgG₂ₐ with siRNA does not react to CD71. Figure 2D shows that the conjugate of Fab' of clone R17 with siRNA (specifically, siRNA against HPRT) competes with clone R17 for binding to CD71. Figure 2E shows that the conjugate of Fab' of clone R17 with siRNA (specifically, siRNA against HPRT) competes only weakly with transferrin (Tf). Figure 2F shows that the conjugate of Fab' of clone R17 with siRNA (specifically, siRNA against HPRT) does not compete with isotype control IgG₂ₐ.

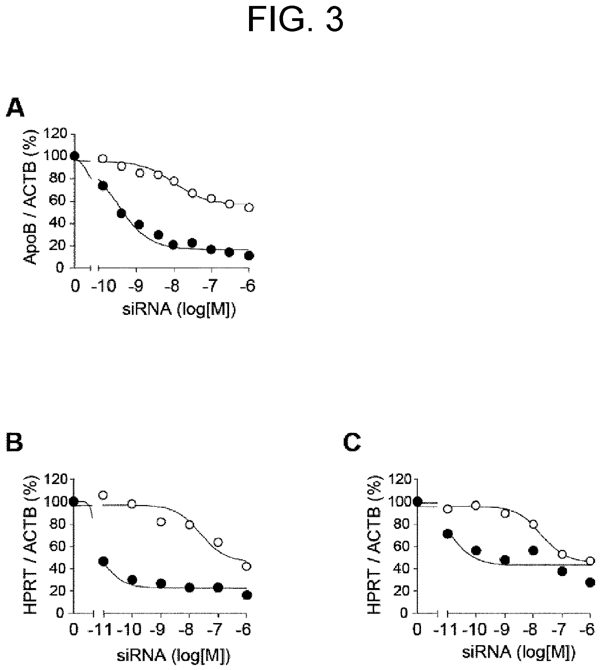

[Figure 3] Figure 3 shows the gene silencing (knock down) by the conjugate of Fab' of clone R17 with siRNA on the cultured cells. Figure 3A shows silencing against ApoB gene expression in the cells, which is a primary hepatocyte weakly expressing CD71, when the conjugate of Fab' of clone R17 with siRNA against ApoB (siApoB) was added to the culture medium of the cells. Figure 3B is a graph showing a silencing against HPRT gene expression in B cells stimulated with anti-IgM which is as an example of a mitogen, when the conjugate of Fab' of clone R17 with siRNA against HPRT (siHPRT) was added to the culture medium. Figure 3C is a graph showing a silencing against HPRT gene expression in T cells stimulated with L-PHA which is as an example of a mitogen, when the conjugate of Fab' of clone R17 with siRNA against HPRT (siHPRT) was added to the culture medium. In Figure 3A, filled circles represent the conjugate of GalNAc-siApoB, and open circles represent the conjugate of clone R17 Fab' with siApoB. In Figures 3B and 3C, filled circles represent the conjugate of clone R17 Fab' with siHPRT, and open circles represent the conjugate of isotype control IgG₂ₐ with siHPRT.

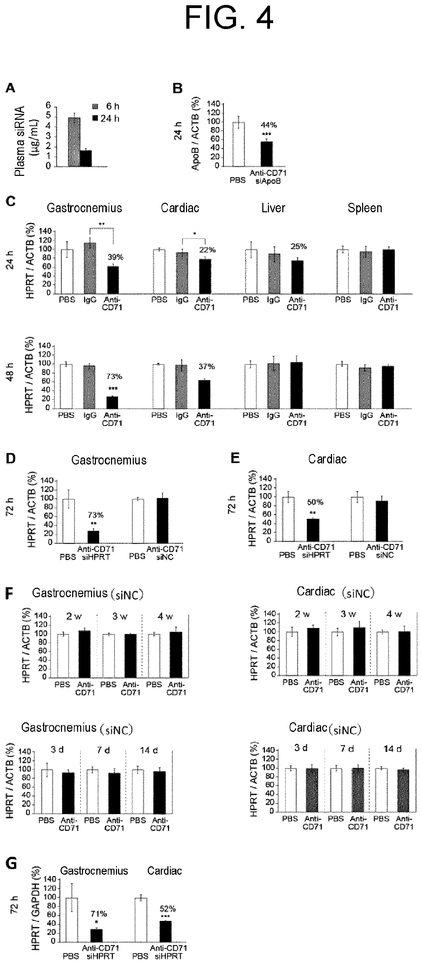

[Figure 4] Figure 4 illustrates in vivo kinetics and function of the antibody-drug conjugate in which the antibody is Fab' of clone R17 and the drug is siRNA. Number (%) in the Figure shows a decrease amount of expression level. Figure 4A shows the amount of the conjugate in plasma at 6 hours and 24 hours after administration. Figure 4B shows the expression level of ApoB in liver at 24 hours after the administration of the conjugate of Fab' of clone R17 with siRNA against ApoB (10 mg/kg bodyweight, intravenous). Figure 4C is a graph showing the silencing against HPRT in each tissue at 24 hours or 48 hours after the administration of the conjugate of Fab' of clone R17 with siRNA against HPRT (10 mg/kg bodyweight, intravenous) (n=4, *: p<0.05, **: p<0.005, ***: p<0.0001, according to student's t-test). Figures 4D and 4E show silencing against HPRT in gastrocnemius muscle (Figure 4D) and cardiac muscle (Figure 4E) at 72 hours after the administration of the conjugate of Fab' of clone R17 with siRNA against HPRT (10 mg/kg bodyweight, intravenous). Figure 4F shows that HPRT silencing does not occur when negative control siRNA was used. In the figure, siNC represents a negative control siRNA. Figure 4G shows that even when the internal reference was changed from β actin (ACTB) used in Figures 4D and 4E to GAPDH, the same results were observed (n=4, *: p<0.05, **: p<0.01, ***: p<0.001, Welch's test).

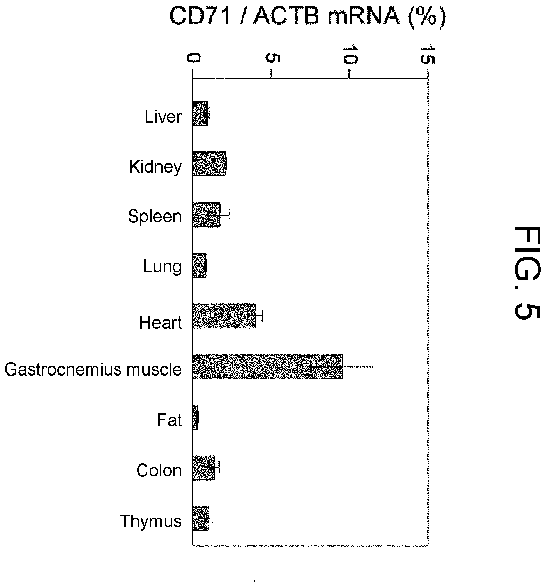

[Figure 5] Figure 5 is a graph showing the expression level of CD71 in each tissue (n=4 for each tissue).

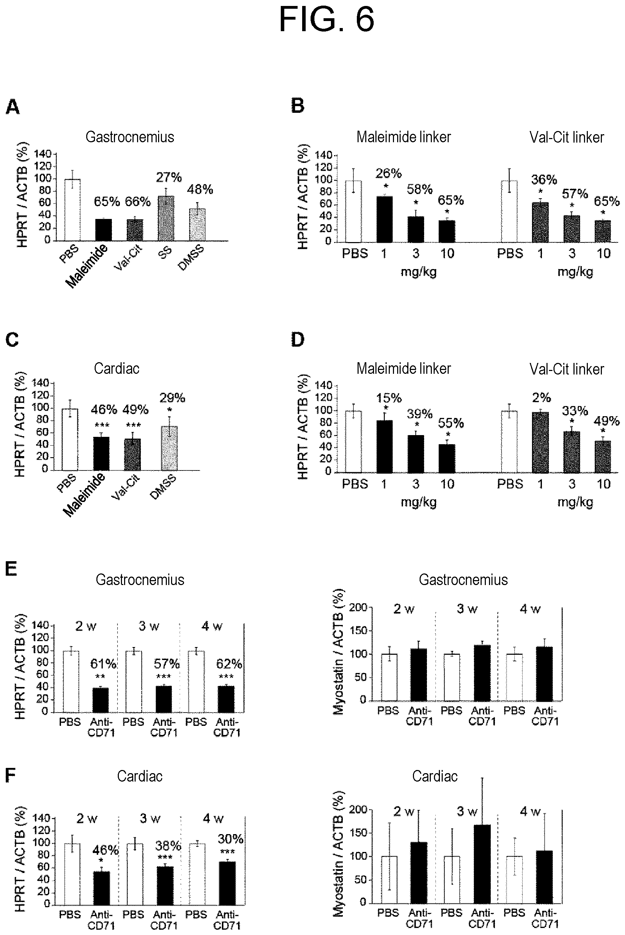

[Figure 6] Figure 6 is a graph showing the effect of silencing depending on the difference of linkers. In Figure 6A, HPRT silencing at 48 hours after administration of the conjugate of Fab' of clone R17 with siRNA against HPRT (10 mg/kg bodyweight, intravenous) are indicated for each type of linkers. The linkers were obtained by reacting the functional group named same as Figure 1A with a thiol group of Fab' according to the scheme shown in Figure 1B. Figure 6B shows the result of comparison between a maleimide linker (non-cleavable) and a Val-Cit linker (cleavable) on the silencing in gastrocnemius muscle at 7 days after administration of the conjugate of Fab' of clone R17 with siRNA against HPRT (10 mg/kg bodyweight, intravenous). Figure 6C shows the comparison of silencing by various linkers in cardiac muscle at 48 hours after administration of the conjugate of Fab' of clone R17 with siRNA against HPRT (10 mg/kg bodyweight, intravenous). Figure 6D shows the result of comparison between a maleimide linker (non-cleavable) and a Val-Cit linker (cleavable) on the silencing in cardiac muscle at 7 days after administration of the conjugate of Fab' of clone R17 with siRNA against HPRT (10 mg/kg bodyweight, intravenous). In Figures 6A and C, n=4, and statistical analysis was conducted by Dunnett's test (*: p<0.05, ***: p<0.001). In Figures 6B and D, n=4, and statistical analysis was conducted by Williams test (*: p<0.05). Figure 6E shows silencing against HPRT or myostatin (negative control) in gastrocnemius muscle at 2 weeks, 3 weeks, and 4 weeks after the administration of the conjugate of Fab' of clone R17 with siRNA against HPRT (10 mg/kg bodyweight, intravenous). Figure 6F shows the silencing against HPRT or myostatin (negative control) in cardiac muscle after the administration as in Figure 6E. In Figures 6E and F, n=4, and statistical analysis was conducted by student's t-test (*: p<0.05, **: p<0.01, ***: p<0.001).

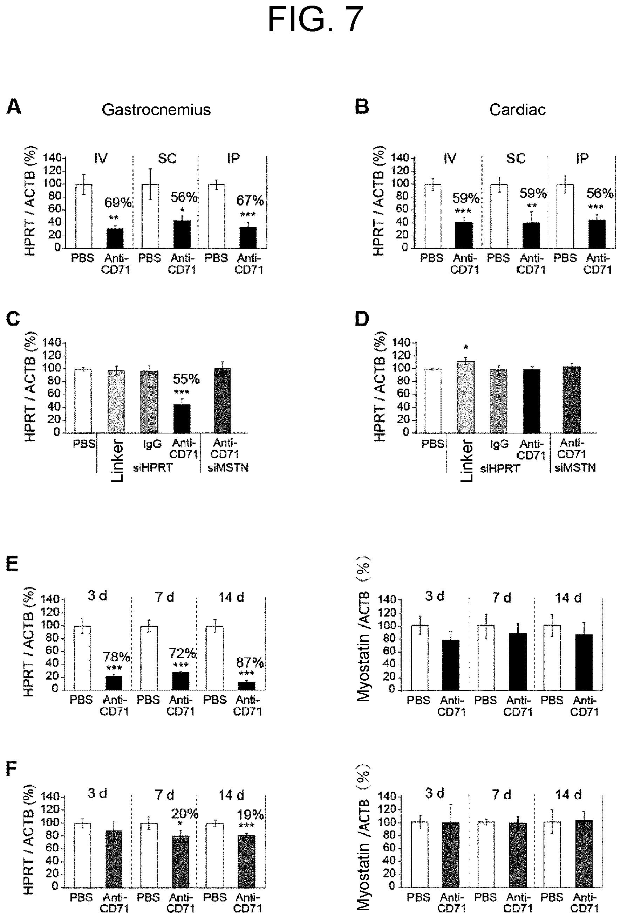

[Figure 7] Figure 7 is a graph showing the effect of difference in administration methods on silencing. In Figure 7, it was verified whether the difference is caused in silencing depending on the administration method by administering the conjugate of Fab' of clone R17 with siRNA against HPRT (10 mg/kg bodyweight) intravenously (IV), subcutaneously (SC) or intraperitoneally (IP). Figure 7A shows the silencing against HPRT in gastrocnemius muscle at 7 days after administration (n=4, *: p<0.05 by Welch test; **: p<0.005 by Welch test; ***: p<0.0001 by student's t-test). Figure 7B shows the silencing against HPRT in cardiac muscle at 7 days after administration. Figure 7C shows the silencing against HPRT in gastrocnemius muscle treated with intramuscular administration of 1 µg of various conjugates or negative control at 7 days after the administration. Figure 7D shows the silencing in the opposite side of the gastrocnemius muscle which is untreated. In Figures 7C and D, n=4, and statistical analysis was conducted by Dunner's test (*: p<0.05; *** p<0.001). Figures 7E and F show the silencing against HPRT or myostatin in gastrocnemius muscle treated with 40 µg of intramuscular administration of the conjugate of Fab' of clone R17 with siRNA against HPRT at 3 days, 7 days or 14 days after the administration, where n=4 and the statistical analysis was conducted by Welch test (***: p<0.001). Figure 7F shows the silencing against HPRT or myostatin after 3 days, 7 days or 14 days in the opposite side of gastrocnemius muscle which is untreated, where n=4 and the statistical analysis was conducted by student's test (*: p<0.05; ***: p<0.001).

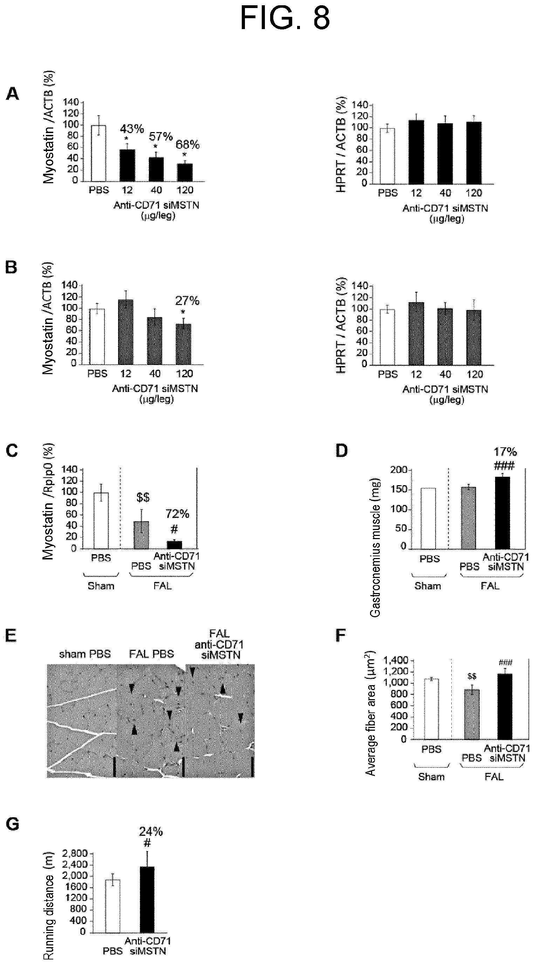

[Figure 8] Figure 8 shows the effect of the conjugate of Fab' of clone R17 with siRNA against myostatin in peripheral artery disease (PAD) mouse model. Figure 8A shows the silencing against myostatin or HPRT by the conjugate administered intramuscularly to gastrocnemius muscle of one leg of a normal mouse. Figure 8B shows the silencing against myostatin or HPRT in gastrocnemius muscle of the other leg which is untreated. In Figures 8A and B, n=4, and statistical analysis was conducted by Williams test (*: p<0.0001). Figure 8C shows the silencing of the conjugate of Fab' of clone R17 with siRNA against myostatin (siMSTN) in PAD mouse with femoral artery ligation (FAL). Figure 8C shows the silencing against myostatin after 4 weeks intramuscular administration once a week of the conjugate of Fab' of clone R17 with siMSTN (20 µg/leg treated with FAL). In Figure 8C, n=3 to 5 and the statistical analysis was conducted by student's t-test ($$: p<0.01 compared to Sham control (sham); #: p<0.05 compared to FAL control). Figure 8D shows the weight of the gastrocnemius muscle in the mouse treated as shown in Figure 8C. In Figure 8D, n=2 to 5 and the statistical analysis was conducted by student's t-test (###: p<0.001 compared to FAL control). Figure 8E shows hematoxylin-eosin stained thin layer sections of the gastrocnemius muscle of mouse treated as shown in Figure 8C. The arrowheads show the muscle fibers and the regenerated muscle fibers are characterized by localized nucleus in the center. The scale bars indicate each 50 µm. Figure 8F shows the average value of the cross-sectional area of muscle fibers in the gastrocnemius muscle shown in Figure 8E. In Figure 8F, the statistical analysis was conducted by student's t-test ($$: p<0.01 compared to Sham control (sham); ###: p<0.001 compared to FAL control). Figure 8G shows the result of the running performance test by treadmill test of the mouse treated as shown in Figure 8C. In Figure 8G, n=12 and the statistical analysis was conducted by student's t-test (#: p<0.05 compared to FAL control).
[Figure 9] Figure 9 shows the gene silencing (knock down) by the conjugate of Fab' of OKT9 antibody with siRNA on cultured human cells. Figure 9 is a graph showing a silencing against HPRT gene expression in human chronic myeloid leukemia cells (K562) that express CD71, when the conjugate of Fab' of OKT9 antibody with siRNA against HPRT (indicated as "CD71-siRNA (HPRT)" in the Figure) added to the culture medium. The conjugate in which Fab' of isotype control IgG₂ₐ was used instead of OKT9 antibody (indicated as "IgG-siRNA (HPRT)" in the figure) was used as a negative control.
[Figure 10] Figure 10 is a graph showing a silencing against MALAT1 in each tissue (n=4) at 48 hours after administration of the conjugate of Fab' of clone R17 with antisense oligo nucleic acid (ASO) against MALAT1 (10 mg/kg bodyweight, intravenous).

### Description of Embodiments

As used herein, the term "subject" means animal (for example, mammal (e.g., primate (e.g., human)).

As used herein, the term "antibody" means an immunoglobulin, and includes polyclonal and monoclonal antibodies. The antibody is preferably a monoclonal antibody.

Source of antibody is not particularly limited, and, for example, the antibody may be a non-human animal antibody (for example, a non-human mammal antibody) and a human antibody. Further, the antibody may be a chimeric antibody or a humanized antibody. The antibodies may be a bispecific antibody. The source of antibody is preferably a humanized antibody or a human antibody.

The antibody is preferably a chimeric monoclonal antibody, a humanized monoclonal antibody and a human monoclonal antibody. It should be noted that a chimeric monoclonal antibody and a humanized monoclonal antibody can be prepared in a manner known per se from a non-human animal antibody.

The antibody has a structure of two heavy chains and two light chains associated with the heavy chains. The heavy chain is composed of a heavy chain variable region (VH), heavy chain constant regions (CH1, CH2 and CH3), and a hinge region located between the heavy chain variable region and the heavy chain constant region. The light chain is composed of the light chain variable region (VL) and the light chain constant region (CL). The heavy chain variable region and light chain variable region each have three complementarity-determining regions (CDRs) which characterize antigen specificity of the antibody. CDRs in the heavy chain are called heavy chain CDR1, heavy chain CDR2 and heavy chain CDR3 from the N-terminal side of the heavy chain, and CDRs in the light chain are called light chain CDR1, light chain CDR2 and light chain CDR3 from the N-terminal side of the light chain.

As used herein, the term "antigen-binding fragment" means a portion of antibody retaining binding ability to an antigen. The antigen-binding fragment may contain a heavy chain variable region or a light chain variable region or both of them of the antibody of the present invention. The antigen-binding fragment may be a chimera or humanized. Examples of the antigen-binding fragment include Fab, Fab', F(ab')₂, Fv, scFv (single chain Fv), diabody, sc(Fv)₂ (single chain (Fv)₂) and a half-molecule type Ig. In the present invention, any of these antigen-binding fragments of an antibody may be used. The method for preparing these antigen-binding fragments of the antibody is not particularly limited, but the fragments may be obtained, for example, by treating the antibody with an enzyme. For example, an antibody may be digested with papain to obtain Fab. Alternatively, an antibody may be digested with pepsin to obtain F(ab')₂, which may be further reduced to obtain Fab'.

As used herein, the term "treating" means a therapy or a prevention.

As used herein, the term "drug" means, for example, a physiologically active substance which is useful in vivo (for example, a protein (for example, an enzyme), a nucleic acid (for example, DNA or a modified nucleic acid), a lipid, a peptide, a carbohydrate, a low molecular weight compound, a metabolite and a secondary metabolite), a contrast agent, a fluorescent dye, or the like (hereinafter sometimes referred to as physiologically active substance or the like). As used herein, the term "drug" is used in the meaning to include not only the physiologically active substance or the like, but also a drug in the form which may release the physiologically active substance or the like in a cell (for example, a prodrug of the physiologically active substance or the like, and a vesicle encapsulating the physiologically active substance or the like). As used herein, the term "physiologically active substance" means a substance that acts on specific physiologic regulatory functions of the living body. By using a contrast agent or a fluorescent dye as a "drug", diagnostic imaging of muscle or the like may be performed.

The "drug" is preferably a physiologically active substance. Further, the physiologically active substance is preferably a "nucleic acid".

As used herein, the term "nucleic acid" include natural nucleic acid such as natural DNA and natural RNA, antisense oligonucleotide (ASO), modified nucleic acid such as modified DNA and modified RNA, artificial nucleic acid, and a combination of these. Examples of the modified nucleic acid include fluorescent dye-modified nucleic acid, biotinylated nucleic acid, and nucleic acid into which a cholesteryl group is introduced. In order to increase the stability, RNA may have 2'-O-methyl modification, or 2'-fluoro modification or 2'-methoxyethyl (MOE) on a base or may have a replacement of a phosphodiester bond in the nucleic acid backbone by a phosphorothioate bond. Examples of the artificial nucleic acid include a nucleic acid in which the 2'-position oxygen atom and 4'-position carbon atom are crosslinked. Examples of such artificial DNA include locked nucleic acid (LNA) which is a bridged DNA in which 2'-oxygen atom and 4'-carbon atom are bridged via a methylene, ENA in which 2'-oxygen atom and 4'-carbon atom are bridged via an ethylene, bridged nucleic acids (BNA) such as BNA^{COC} in which 2'-oxygen atom and 4'-carbon atom are bridged via -CH₂OCH₂- and BNA^{NC} in which 2'-oxygen atom and 4'-carbon atom are bridged via -NR-CH₂- {wherein R is methyl or a hydrogen atom}, cMOE in which 2'-oxygen atom and 4'-carbon atom are bridged via -CH₂(OCH₃)-, cEt in which 2'-oxygen atom and 4'-carbon atom are bridged via -CH₂(CH₃)-, AmNA in which 2'- and 4'-carbon atoms are bridged via amido, scpBNA in which 2'-oxygen atom and 4'-carbon atom are bridged via methylene, and cyclopropane is formed at the 6'-position; and peptide nucleic acid (PNA) in which a polymer composed of amide-linked N-(2-aminoethyl) glycine is used instead of deoxyribose or ribose as backbone. Examples of RNA include artificial RNA for gene silencing such as siRNA and shRNA, micro RNA (miRNA), non-coding RNA such as aptamers, and natural RNA such as mRNA. These RNAs may be modified to stabilize in vivo.

As used herein, the term "conjugate" means a conjugation body in which two substances are covalently linked. In the conjugate, the two substances may be linked directly or may be linked via a linker. In the present invention, one of the two substances is an antibody or antigen-binding fragment thereof, and the other is a drug (for example, a physiologically active substance). In the present invention, the linker may be a cleavable linker or may be a non-cleavable linker.

As used herein, the term "antibody-drug conjugate" means a conjugate of an antibody with a drug (ADC). The affinity to an antigen is imparted to a drug by linking an antibody with the drug, thereby it may increase the efficiency of delivering the drug to a target site in vivo. As used herein, the antibody-drug conjugate is used in the sense that includes a conjugate of an antigen-binding fragment of an antibody with a drug.

The term "CD71" is known as a transferrin receptor having a function of incorporating iron into a cell. CD71 is expressed in hepatocytes. Further, although not expressed in lymphocytes of a normal resting state, CD71 is expressed in lymphocytes when activated with mitogen or the like. Moreover, CD71 is highly expressed in muscle (for example, cardiac muscle and gastrocnemius muscle). Apo-transferrin (i.e., non-iron conjugate) binds to two Fe³⁺ ions to form holo-transferrin (i.e., iron conjugate) which binds to CD71. The complex of CD71 and holo-transferrin enters the cell by receptor-mediated endocytosis. CD71 and transferrin are separated under the environment of endosome, and transferrin enters the cell, while CD71 is recycled to the cell membrane. Accordingly, transferrin is believed to enter the cell by the appropriate binding to and dissociation from CD71. Examples of CD71 include human CD71 (for example, having the sequence set forth in accession number XM 011513112 of GenBank), mouse CD71 (for example, having the sequence set forth in accession number NM 011638 of GenBank). The cell may be a cell expressing CD71 (for example, eukaryotic cell, for example, mammalian cell, for example, primate cell, for example, human cell), or a tissue or organ including the cell. CD71 expressed on the cell may be endogenous CD71 or exogenous CD71. CD71 expressed in a cell may be those induced to be expressed.

As used herein, the term "anti-CD71 antibody" means an antibody recognizing CD71, or an antibody binding to CD71. The anti-CD71 antibody is preferably an antibody recognizing human CD71. The anti-CD71 antibody is preferably a monoclonal antibody, especially, more preferably an anti-CD71 chimeric monoclonal antibody, an anti-CD71 humanized monoclonal antibody, or an anti-CD71 human monoclonal antibody.

As used herein, the term "human monoclonal antibody" means any antibody in which the sequences of variable and constant domains are human sequences. The term includes an antibody having a sequence derived from human genes, while having changes to perform reduction of possible immunogenicity, increase of affinity, or removal of cysteines that might cause undesirable folding or the like. The term also includes such an antibody which is prepared recombinantly in non-human cells and can be subjected to glycosylation which is not typical of human cells. These antibodies can be prepared in various forms as described below.

As used herein, the term "chimeric monoclonal antibody" means a monoclonal antibody formed by combining antibody regions from two or more different species. Examples of the chimeric monoclonal antibody include a monoclonal antibody formed by combining a variable region of mouse antibody with a constant region of human antibody.

As used herein, the term "chimeric human monoclonal antibody" includes a monoclonal antibody containing VH and VL domains of non-human mammalian species antibody and CH and CL domains of human antibody.

One or more CDRs of chimeric antibody may be derived from human antibodies. In one example, CDRs derived from human antibodies may be combined with CDRs derived from antibodies from non-human mammals, such as mouse and rat. In another example, all CDRs may be derived from human antibodies. In another example, CDRs derived from a plurality of human antibodies may be combined in a chimeric antibody. For example, the chimeric antibody may include CDR1 of light chain of a first human antibody, CDR2 of light chain of a second human antibody, and CDR3 of light chain of a third human antibody, and CDRs of heavy chain derived from one or more other antibodies.

As used herein, the term "humanized monoclonal antibody" refers to a monoclonal antibody from a non-human mammal, in which amino acid residues characteristic to the sequence of the non-human mammal antibody are substituted with residues found at the corresponding positions of human antibody. It is believed that this "humanized" process reduces the immunogenicity of the resulting antibody in humans. Non-human mammalian-derived antibodies can be humanized using well-known technologies in the art (for example, technology described in Winter et al., Immunol. Today, 14:43-46 (1993)). Humanized monoclonal antibodies can be prepared by engineering using, for example, recombinant DNA techniques (for example, techniques described in WO92/02190, and US Patents Nos. 5,530,101, 5,585,089, 5,693,761, 5,693,792, 5,714,350, and 5,777,085) in which CH1, CH2, CH3, hinge domains and/or framework domains of the antibodies from non-human mammals are substituted with the corresponding human sequences. As used herein, the term "humanized monoclonal antibody" includes, within its meaning, a chimeric human monoclonal antibody and a CDR-grafted antibody. The CDR-grafted antibody of the present invention can be obtained by substituting CDRs of VH and VL of a human antibody with CDRs of VH and VL of a non-human animal antibody, respectively.

As used herein, "muscle" means striated muscle and smooth muscle. The striated muscle means muscle with striated structure, and examples thereof include skeletal muscle and cardiac muscle. The smooth muscle means muscle without striated structure and examples thereof include visceral muscle.

As used herein, "vascular endothelial cell" means a cell lining the blood vessel and usually a thin, flat cell. Vascular endothelial cells control passages of substances and leukocyte to inside and outside of blood vessel. Thus, the drug administered intravascularly is necessary to pass through the layer of vascular endothelial cells in order to move into tissue parenchyma.

According to the present invention, a conjugate of an anti-CD71 antibody with a drug may be provided. Further, according to the present invention, a conjugate of an antigen-binding fragment (e.g., Fab') of an anti-CD71 antibody with a drug may be provided. Furthermore, according to the present invention, a conjugate of Fab' of the anti-CD71 antibody with a nucleic acid may be provided. These conjugates may be effectively delivered to, for example, muscle ((e.g., cardiac muscle and gastrocnemius muscle). Further, these conjugates may be administered, for example, by intravenous administration, intramuscular administration, subcutaneous administration, or intraperitoneal administration. These conjugates are administered intravenously or intramuscularly, thereby effectively delivered to muscle (for example, cardiac muscle and gastrocnemius muscle), and further delivered into the cell. These conjugates may be suitably used to deliver a drug into a cell expressing CD71 (hereinafter sometimes referred to as "CD71 positive"), for example, muscle cells, cancer cells, immune cells (e.g. lymphocytes such as B cells and T cells) and hepatocytes. In addition, these conjugates can be applied *ex vivo* to CD71-positive cells and tissues. A person skilled in the art can determine whether the cell is CD71-positive, for example, by flow cytometry using anti-CD71 antibodies which are used in the preparation of the conjugate.

The incorporation of transferrin into cells by CD71 is performed by endocytosis, and even, for example, a vesicle having a particle size of about 300 nm or a particle size of 300 nm or less may be incorporated into the cell by endocytosis. Further, even when a vesicle is a macromolecule, the vesicle may be incorporated into the cell together with CD71. Accordingly, in one aspect of the present invention, there is provided a vesicle having a particle size of about 300 nm or a particle size of 300 nm or less, wherein the vesicle having an anti-CD71 antibody or an antigen-binding fragment thereof on the surface. The vesicle may contain a drug therein. In the specification, unless otherwise specified, the vesicle containing a physiologically active substance therein is also called a "drug". The type of vesicle is not particularly limited, and examples thereof include a liposome using lipid molecules and a Lipid Nanoparticle (LNP). The grain size of the vesicle is not particularly limited, and the upper limit thereof may be typically 300 nm, preferably 250 nm, more preferably 200 nm. The lower limit of the particle size of the vesicles is not particularly limited, and the lower limit thereof may be 30 nm, 40 nm, 60 nm or 80 nm.

In the present invention, an anti-CD71 antibody or an antigen-binding fragment thereof may be linked with a drug via a linker. As used herein, the linker, which the conjugate of the present invention may have, represents a portion which links between an anti-CD71 antibody or an antigen-binding fragment thereof and a drug.

When the conjugate of the present invention has a linker, as illustrated in the examples described below, the linker may be a cleavable linker or a non-cleavable linker. Here, "cleavable" means that the linker is cleavable in an intracellular environment (for example, low pH environment inside endosome or intracellular reducing environment), and "non-cleavable" means that the linker does not cleave or substantially cleave in the intracellular environment. That is, in the present invention, any linker may be used as long as the linker is not so actively decomposed at the outside of the cell that the drug cannot reach into the cell. The linker is not particularly limited, and a variety of linkers may be used.

In the present invention, either of cleavable linker and non-cleavable linker may be used as a linker.

Examples of the cleavable linker include a linker having a -S-S- bond in the structure where the -S-S- bond is a bond cleaved under intracellular reducing environment (for example, SS linker, DMSS linker), a linker having a hydrazone bond in the structure where the hydrazone bond is a bond cleaved by low pH in endosome, a linker having an ortho ester bond in the structure, and a linker having a peptide bond being cleaved by cathepsin B in the structure (for example, the linker having a valine-citrulline bond in the molecule (Val-Cit linker)). These linkers may be preferably used in the present invention.

Examples of the cleavable linker, in addition to the ones above, include a linker having a carbohydrate chain in the structure where the carbohydrate chain is cleaved by carbohydrate chain-degrading enzyme such as glucuronidase. These linkers may be preferably used in the present invention.

Examples of the non-cleavable linkers include a linker which has a thiol-reactive group that is a maleimide group and does not have cleavable binding sites that may be cleaved in an intracellular environment (for example, low pH environment inside endosome or intracellular reducing environment) in the structure, which is herein sometimes referred to as a maleimide linker. The maleimide linker may be preferably used in the present invention. As used herein, the maleimide linker is a linker having a maleimide group and distinguished from cleavable linker (for example, DMSS linker, Val-Cit linker) and any other cleavable linker (for example, SS linker).

In an aspect of the present invention, the linker is preferably a maleimide linker, a Val-Cit linker, an SS linker, and a DMSS linker. In an aspect of the present invention, it is preferred to use the linker for example, represented by one of the formulae (I) to (IV) described below.

It is preferred that the anti-CD71 antibody or the antigen-binding fragment thereof used in the present invention may bind to CD71 stronger than transferrin in blood or may bind to CD71 at a different site from the site transferrin binds to. These anti-CD71 antibody or antigen-binding fragment thereof may bind to CD71 in vivo, regardless of the presence of blood transferrin.

The anti-CD71 antibody or antigen-binding fragment thereof, when bound to CD71, may be incorporated into cells together, by spontaneous intracellular incorporation effect of CD71.

Evaluation of whether the anti-CD71 antibody or the antigen-binding fragment thereof may bind to CD71 stronger than blood transferrin or not can be carried out, for example, by comparing the binding activity to CD71 of the anti-CD71 antibody or the antigen-binding fragment thereof with that of the blood transferrin. The comparison of the binding activity to CD71 of the antibody or the antigen-binding fragment thereof with that of transferrin can be performed, for example, by competitive binding activity evaluation. More specifically, the well plates incubated with CD71 are washed, and then blocked with D-PBS(-) containing 1% bovine serum albumin (BSA). After further washing, the anti-CD71 antibody or the antigen-binding fragment thereof and transferrin are incubated in the well. After washing, labeling is performed in the well with a suitable label (e.g., fluorescent label). Then, from the detected intensities, the competitive binding activity can be evaluated.

Whether the anti-CD71 antibody or the antigen-binding fragment thereof binds to CD71 at a different site from that of transferrin binds can be evaluated, for example, in the evaluation of the competitive binding activity described above, by labeling the anti-CD71 antibody or the antigen-binding fragment thereof and transferrin each with distinguishable different labels, and evaluated the competitive binding activities from the detected intensities for each or as a whole. In the evaluation, for example, in the case of using the anti-CD71 antibody or the antigen-binding fragment thereof and the transferrin each labeled with distinguishable fluorescent different labels, when combined fluorescence is detected, it can be said that both the anti-CD71 antibody or the antigen-binding fragment thereof and transferrin are bound to CD71, and thus it can be evaluated as they are bound to CD71 at different sites from each other.

The anti-CD71 antibody is preferably a clone R17 217.1.3 (Bio X Cell), OKT9 antibody (manufactured by Bio X Cell: BE0023 (OKT9)), or an anti-human CD71 antibody (5E9C11). In an aspect, the anti-CD71 antibody is preferably a chimeric monoclonal antibody of clone R17 217.1.3, a humanized monoclonal antibody of clone R17 217.1.3, or a human monoclonal antibody of clone R17 217.1.3, a chimeric monoclonal antibody of OKT9 antibody (manufactured by Bio X Cell: BE0023), a humanized monoclonal antibody of OKT9 antibody (Bio X Cell: BE0023), or a human monoclonal antibody of OKT9 antibody (manufactured by Bio X Cell: BE0023), a chimeric monoclonal antibody of anti-human CD71 antibody (5E9C11), a humanized monoclonal antibody of anti-human CD71 antibody (5E9C11), or a human monoclonal antibody of anti-human CD71 antibody (5E9C11); an antibody comprising a heavy chain variable region and a light chain variable region having the same amino acid sequence as any of these antibodies; or an antibody comprising heavy chain CDRs l to 3 and light chain CDRs l to 3 having the same amino acid sequence as any of these antibodies. The amino acid sequence of CDR can be determined according to Kabat numbering. The antigen-binding fragment used in the present invention is preferably Fab' of any of those antibodies.

The anti-CD71 antibody or antigen-binding fragment thereof used in the present invention is not particularly limited, but it is preferred that the amino acid sequence of CDR thereof has substantial identity (homology) to the corresponding CDR of the aforementioned clone R17 217.1.3 (manufactured by Bio X Cell), OKT9 antibody (manufactured by Bio X Cell: BE0023) or a chimeric monoclonal antibody, humanized monoclonal antibody or human monoclonal antibody) of anti-human CD71 antibody (5E9C11). Further, the anti-CD71 antibody or antigen-binding fragment thereof used in the present invention is preferably the antibody which competes for binding to human CD71 with the chimeric monoclonal antibody, or humanized monoclonal antibody or human monoclonal antibody of OKT9 antibody (manufacture by Bio X Cell: BE0023) or anti-human CD71 antibody (5E9C11), or an antigen-binding fragment thereof; or the antibody having an epitope completely or partially identical to (or the antibody having an overlapped epitope with) that of any of antibodies described above in binding to CD71, or an antigen-binding fragment thereof; or an antibody containing the amino acid sequence having addition, insertion, deletion, or substitution of one to several amino acids in the amino acid sequence of these antibodies, or an antigen-binding fragment thereof.

The antibody or antigen binding fragment thereof having substantial identity (homology) to the CDR of OKT9 antibody (manufactured by Bio X Cell: BE0023) is preferably an antibody having a heavy chain variable region containing a heavy chain CDR1 having an amino acid sequence represented by SEQ ID NO: 26, a heavy chain CDR2 having an amino acid sequence represented by SEQ ID NO: 27, and a heavy chain CDR3 having an amino acid sequence represented by SEQ ID NO: 28, and a light chain variable region containing a light chain CDR1 having an amino acid sequence represented by SEQ ID NO: 29, a light chain CDR2 having an amino acid sequence represented by SEQ ID NO: 30, and a light chain CDR3 having an amino acid sequence represented by SEQ ID NO: 31, or an antigen-binding fragment thereof; or an antibody having a heavy chain variable region containing an amino acid sequence represented by SEQ ID NO: 24 and a light chain variable region containing an amino acid sequence represented by SEQ ID NO: 25 or an antigen-binding fragment thereof. It should be noted that, in the present specification, these CDR can be found by applying them to the method described in Kabat et.al, Ann. NY Acad Sci 190:382-93 (1971) or the database of amino acid sequences of antibody created by Kabat et al, ([Sequence of Proteins of Immunological Interest] US Dept. Health and Human Services, 1983) and examining the homology (for example, the heavy chain variable region of OKT9 can be referred to GenBank registration number:AAA51064.1 and the light chain variable region of OKT9 can be referred to GenBank registration number:AAA51130.1).

The term "substantial identity" means that sequence identity between two amino acid sequences is at least 700, 75% or 800, preferably at least 90% or 950, more preferably at least 970, 980 or 99%. Further, in certain embodiments, the amino acid at position where the sequences are not identical may be substituted by a conservative amino acid.

The term "% sequence identity" means the residue% of identical between two amino acid sequences when the two amino acid sequences are aligned so that the identity of amino acid residues (or conservative amino acids) become maximum.

The identity of amino acid sequences can be determined by any method known to those skilled in the art. For example, the identity of amino acid sequences can be determined by the algorithm of Karlin and Altshul (Proc. Natl. Acad. Sci. USA 87:2264-2268, 1990 and Proc. Natl. Acad. Sci. USA 90:5873-5877, 1993). More specifically, the identity can be determined by a program using the algorithm such as BLAST program (J. Mol. Biol. 215:403-410, 1990). The program for determining the identity of amino acid sequences is available, for example, at the web site on the internet of the US National Center for Biotechnology Information.

The conjugate of the present invention is preferably a conjugate, wherein
(i) the anti-CD71 antibody or the antigen-binding fragment thereof is a chimeric monoclonal antibody of clone R17 217.1.3, a humanized monoclonal antibody of clone R17 217.1.3 or a human monoclonal antibody of clone R17 217.1.3, or an antigen-binding fragment (preferably, Fab') thereof;
(ii) the drug is a physiologically active substance (for example, a nucleic acid (preferably, mRNA, siRNA or ASO (more preferably, siRNA or ASO)); and
(iii) the anti-CD71 antibody or the antigen-binding fragment thereof and the drug are linked via a linker (preferably, a maleimide linker, a Val-Cit linker, an SS linker, or a DMSS linker).

The conjugate of the present invention is preferably a conjugate, wherein
(i) the anti-CD71 antibody or the antigen-binding fragment thereof is
   (i-a) an antibody including a heavy chain variable region containing a heavy chain CDR1 having an amino acid sequence represented by SEQ ID NO: 26, a heavy chain CDR2 having an amino acid sequence represented by SEQ ID NO: 27 and a heavy chain CDR3 having an amino acid sequence represented by SEQ ID NO: 28, and a light chain variable region containing a light chain CDR1 having an amino acid sequence represented by SEQ ID NO: 29, a light chain CDR2 having an amino acid sequence represented by SEQ ID NO: 30 and a light chain CDR3 having an amino acid sequence represented by SEQ ID NO: 31, or an antigen-binding fragment thereof;
   (i-b) an antibody (preferably, human antibody) containing a heavy chain variable region having an amino acid sequence represented by SEQ ID NO: 24 and a light chain variable region having an amino acid sequence represented by SEQ ID NO: 25, or an antigen-binding fragment thereof; (i-c) an antibody (preferably, human antibody) competing for binding to CD71 with the antibody (i-a) or (i-b) above, or an antigen-binding fragment thereof;
   (i-d) an antibody (preferably, human antibody) where the epitope of the antibody is completely or partially identical to that of the antibody (i-a) or (i-b) above in binding to CD71, or an antigen-binding fragment thereof; or
   (i-e) an antibody (preferably, human antibody) containing the amino acid sequence having addition, insertion, deletion, or substitution of one to several amino acids in the amino acid sequence of the antibody (i-a) described above, or an antigen-binding fragment thereof.
(ii) the drug is a physiologically active substance (for example, a nucleic acid (preferably, mRNA, siRNA or ASO (more preferably, siRNA or ASO)); and
(iii) the anti-CD71 antibody or the antigen-binding fragment thereof and the drug are linked via or by a linker (preferably, a maleimide linker, a Val-Cit linker, a SS linker, or a DMSS linker).

The present invention may provide a composition for delivering a drug to muscle (including, for example, skeletal muscle, cardiac muscle, and neuromuscular junction of these), the composition contains a conjugate of an anti-CD71 antibody or an antigen-binding fragment thereof with a drug. The composition may be administered intravenously, subcutaneously, intraperitoneally, or intramuscularly. The anti-CD71 antibody or the antigen-binding fragment is preferably Fab' of the anti-CD71 antibody.

According to the examples described below, the conjugate of an antigen-binding fragment Fab' of anti-CD71 antibody, with a drug was incorporated into a cell expressing CD71. Thus, the conjugate of antigen-binding fragment of anti-CD71 antibody with a drug may bind to CD71 in vivo and be incorporated into CD71-expressing cell.

According to the present invention, a conjugate of Fab' of an anti-CD71 antibody with a drug may be provided. Fab' obtained by reducing F(ab')₂ has a thiol group. Thus, Fab' and a drug may be linked via S atom (sulfur atom) contained in the thiol group of Fab'.

Fab' may be obtained from antibodies by a method known per se. For example, Fab' may be obtained by subjecting an antibody to pepsin degradation to obtain F(ab')₂ and further reducing F(ab')₂ with a reducing agent such as cysteamine to obtain Fab'.

In one aspect of the present invention, in the conjugate of Fab' with a drug, the drug may be linked to the Fab' via a linker or without a linker. With regard to the conjugate of Fab' with a drug, the introduction of a linker and the preparation of the conjugate may be performed according to the method well-known to those skilled in the art.

When linking Fab' and a drug via a linker, from the viewpoint of the convenience of the reaction and the biological toxicity of the resulting conjugate, the linking may be performed, for example, by reacting a thiol group in Fab' with a thiol-reactive group in the linker introduced into the drug (for example, see Figure 1B) .

According to the present invention, Fab' is excellent in balance between the binding affinity to the membrane protein for recycling and the dissociation property in endosome. Accordingly, the conjugate of an antigen-binding fragment Fab' with a drug may be preferably used in the delivery of a drug into cells.

According to the present invention, a conjugate of Fab' of an anti-CD71 antibody with siRNA is provided. The size of siRNA is about 15 kDa, thus it is rapidly removed from the blood by renal clearance. However, in the form of a conjugate with Fab', siRNA can exist more stably in blood.

The present invention provides a conjugate of an anti-CD71 antibody or an antigen-binding fragment thereof (for example, Fab') with siRNA or ASO against myostatin. The conjugate of an anti-CD71 antibody or an antigen-binding fragment thereof (for example, Fab') with siRNA or ASO against myostatin reduces the expression level of myostatin in muscle or cardiac muscle, thereby allows to increase quantity of muscle. The present invention provides a medicament for use in increasing quantity of muscle in a subject in need thereof, the medicament containing the conjugate of an anti-CD71 antibody or an antigen-binding fragment thereof (for example, Fab') with siRNA or ASO against myostatin. In this aspect, the medicament may be administered, for example, intravenously, subcutaneously, intraperitoneally or intramuscularly. Examples of the siRNA against myostatin include siRNA having a sense strand having a nucleotide sequence represented by SEQ ID NO: 15 (or SEQ ID NO: 5) and an antisense strand having a nucleotide sequence represented by SEQ ID NO: 16 (or SEQ ID NO: 6). The siRNA against myostatin can be used as RNA described in 3) of (2) in Example 1. Examples of the myostatin include human myostatin (for example, a human myostatin having the sequence set forth in accession number NM_005259.2 of GenBank).

As used herein, the antisense or antisense strand refers to a DNA fragment or RNA fragment having a sequence complementary to the certain sequence of DNA or RNA, and examples thereof include one capable of complementarily binding to mRNA. Thus, the antisense strand in the siRNA means an RNA strand complementarily binds to mRNA to be targeted.

As used herein, the sense strand refers to one strand of the double-stranded nucleic acid and which is not an antisense strand.

The siRNA in the present invention may be siRNA having a modification for stabilization. Examples of the modification for stabilization include 2'-O-methyl modification and 2'-fluoro modification. RNA may have alternative modification of 2'-O-methyl modification and 2'-fluoro modification to consecutive bases thereof.

In the present invention, the drug may be siRNA. In the present invention, the thiol-reactive group may be introduced to the sense strand or antisense strand of siRNA. In an aspect of the present invention, the thiol-reactive group may be introduced to the sense strand of siRNA (for example, 3' end of the sense strand). By doing so, it is considered that the antisense strand of the siRNA can be easily attached to the mRNA of a gene to be silenced in the cytoplasm causing RNA interference (RNAi).

In the present invention, the drug may be an antisense oligo nucleic acid (ASO). In the present invention, the thiol-reactive group may be introduced into the aminated 5' or 3' end of the antisense oligo nucleic acid. In an aspect of the present invention, the thiol-reactive group may be introduced at the 5' end of ASO. In an aspect of the present invention, ASO and the thiol-reactive group may be linked via a spacer, for example, a spacer consisting of a nucleic acid of one to several bases.

For therapy for cancer, drug may be a substance having anti-cancer activity. Examples of the substance having anti-cancer activity include a substance having at least one activities of reduction of tumor size (delay or stop of tumor enlargement), inhibition of metastasis of tumor, inhibition (delay or stop) of tumor growth, and alleviation of one or more symptoms related to cancer. Specific examples of the substance having anti-cancer activity include, but are not limited to, a toxin, an anti-cancer agent, and a radioisotope.

Examples of the toxin include Pseudomonas exotoxin (PE) or cytotoxic fragment thereof (for example, PE38), diphtheria toxin, and ricin A.

Examples of the anti-cancer agent include low molecular weight compounds such as adriamycin, daunomycin, mitomycin, cisplatin, vincristine, epirubicin, methotrexate, 5-fluorouracil, aclacinomycin, nitrogen mustard, cyclophosphamide, bleomycin, daunorubicin, doxorubicin, vincristine, vinblastine, vindesine, tamoxifen, and dexamethasone, and proteins such as cytokines activating immunocompetent cells (e.g., human interleukin-2, human granulocyte-macrophage colony-stimulating factor, human macrophage colony-stimulating factor, human interleukin-12).

Examples of the radioisotope include ³²P, ¹⁴C, ¹²⁵I, ³H, ¹³¹I, ²¹¹At and ⁹⁰Y.

Other examples of the substance having anti-cancer activity include substances which alter gene expression in immune cells and/or tumor cells. By altering gene expression in immune cells and/or tumor cells, it is possible to maintain or enhance the action of immune cells to tumor cells, tumor tissues and the like. Examples of the substance which alter gene expression in immune cells include nucleic acids such as siRNA and ASO which are capable of silencing PD1 gene, endogenous T cell receptor gene, or the like. Examples of the substance which alter gene expression in tumor cells include nucleic acids such as siRNA and ASO which are capable of silencing immune checkpoint substances such as PD-L1 gene and PD-L2 gene.

### (Production method)

An antibody may be obtained according to conventional manner, as would be recognized by a person skilled in the art, by immunizing an animal with an antigen. A monoclonal antibody can be obtained according to conventional manner, as would be recognized by a person skilled in the art, by fusing a spleen cell of an animal immunized with antigen with myeloma to form a hybridoma, and cloning the hybridoma which produces an antibody that binds to an antigen. Thus, a monoclonal antibody can be obtained as an antibody produced from hybridoma. The anti-CD71 antibody can be obtained by using CD71 as the antigen. The anti-CD71 antibody may be obtained by immunizing animals with virus-like particles (VLP) displaying CD71. The anti-CD71 antibody may be obtained by further selecting the antibody not binding to VLP which does not display CD71, in order to remove the antibodies binding to VLP. The anti-CD71 antibody may be obtained by immunizing an animal with a cell displaying CD71. The anti-CD71 antibody may be obtained by further selecting the antibody not binding to cell which does not display CD71, in order to remove the antibodies binding to the cell. The antigen-binding fragment of the antibody may be obtained by treating the resulting antibody with a method well-known to those skilled in the art.

An antibody that competes with a certain antibody (desired antibody) for binding to CD71 may be obtained by competitive assay. It can be regarded as an antibody that competes with the desired antibody for binding to CD71, when the antibody blocks in a competitive assay the binding of the desired antibody to CD71, for example, at least 200, preferably at least 20 to 500, more preferably at least 50%, 60%, 70%, 80% or 90%. The antibody that competes with the desired antibody can be determined by a cross-blocking assay, preferably by a competitive ELISA assay. In the cross-blocking assay, CD71 is applied to, for example, a microtiter plate, and incubated with the addition of the labeled antibody of interest to form a bond between the antigen and the antibody of interest. Thereafter, a candidate competitive antibody is added to the well, the obtained mixture is incubated and washed, and then, by quantifying the binding amount of the antibody of interest (residual labelled amount), whether or not the candidate competitive antibody competes with the antibody of interest can be determined.

The antibody which binds to epitope completely or partially identical to that certain antibody binds for binding to CD71 can be obtained by competitive assay as described above. The antibody which binds to epitope completely or partially identical to that certain antibody binds to for CD71 may also be obtained by hydrogen-deuterium exchange mass spectrometry (HDX MS). In the hydrogen-deuterium exchange mass spectrometry, protein binding surface can be identified by detecting the change in mass caused by the replacement of deuterium in a solution with an amide hydrogen of the protein surface (the replacement hardly occurs in the protein binding surface).

A person skilled in the art can prepare a conjugate of an antibody with a drug by using a method well-known as a method for preparing an antibody-drug conjugate (ADC). The same applies to a conjugate of an antigen-binding fragment of antibody with a drug.

The present invention provides a method for producing a conjugate of an antigen-binding fragment Fab' with a drug, comprising introducing a group for introducing a thiol-reactive group to a drug or a carbon chain having a substituent bound to the drug. The present invention provides a method for producing a conjugate of an antigen-binding fragment Fab' with a drug, comprising introducing a group for introducing a thiol-reactive group to a drug or a carbon chain having a substituent bound to the drug, and reacting a thiol group of an antigen-binding fragment Fab' with the drug or the thiol-reactive group introduced to the drug.

When the drug is ASO, the thiol-reactive group, the group for introducing a thiol-reactive group, or the carbon chain having a substituent may be introduced by linking via a spacer (e.g., a spacer consisting of a nucleic acid of one to several bases) introduced to the

### ASO.

The conjugate of the present invention having a linker can be produced by a known method per se. For example, it can be produced by linking a drug with a carbon chain containing a substituent (preferably a carbon chain having an amino group at the end), then linking the amino group to a group for introducing a thiol-reactive group, and further reacting the thiol-reactive group with an anti-CD71 antibody or a binding fragment thereof.

The carbon number of the carbon chain having a substituent described above may be 1 to 10, preferably 2 to 8, more preferably 4 to 6, further preferably 6.

In the carbon chain having a substituent, the substituent may be, for example, an amino group or a thiol group, and may be preferably an amino group.

Among the carbon chain having a substituent described above, it may be especially preferred to use an alkyl chain of carbon number 6 having an amino group at the end (herein sometimes referred to as C6 amino chain).

The binding between a drug and the carbon chain having a substituent may be carried out, when the drug is a nucleic acid, through a nucleic acid synthesis reaction by the phosphoramidite method using commercially available 3' or 5'-amino-modified carrier or amidite reagent (e.g., without limiting to, 3'-PT-Amino-Modifier C6 CPG, or 5'-DMS(O) MT-Amino-Modifier C6 (Glen Research Co.)).

When using, as a carbon chain having a substituent, a carbon chain having an amino group at the end, the amino group may be bound to a group for introducing a thiol-reactive group in the next step.

The thiol-reactive group means a functional group having reactivity with a specific site (thiol group) of an anti-CD71 antibody or a binding fragment thereof. Examples of the thiol-reactive group include a maleimide group, a bromoacetamide group, a pyridyldithio group, a vinyl sulfone group, and an iodoacetamide group.

The group for introducing a thiol-reactive group means a reagent having a thiol-reactive group. Examples of the reagent include maleimide caproyl (mc); maleimide caproyl-p-aminobenzyl carbamate; maleimide caproyl-peptide-aminobenzyl carbamate (for example, maleimide caproyl-L-phenylalanine-L-lysine-p-aminobenzyl carbamate and maleimide caproyl-L-valine-L-citrulline-p-aminobenzyl carbamate (vc)); N-maleimide caproyl-valyl-citrullyl-p-aminobenzyl carbamate p-nitrophenyl ester (mc-Val-Cit-PABC-PNP); N-succinimidyl 3-(2-pyridyldithio)propionate (N-succinimidyl 4-(2-pyridyldithio)pentanoate; N-succinimidyl 4-methyl-4-(2-pyridyldithio)pentanate; 4-succinimidyl-oxycarbonyl-2-methyl-2-(2-pyridyldithio)-toluene (SMPT); N-succinimidyl 3-(2-pyridyldithio)propionate (SPDP); N-succinimidyl 4-(2-pyridyldithio)butyrate (SPDB); 2-iminothiolane; S-acetylsuccinic anhydride; disulfide benzyl carbamate; carbonate; hydrazone linker; N-(α-maleimide acetoxy)succinimide ester; N-[4-(p-azidosalicylamide)butyl]-3'-(2'-pyridyldithio)propionamide (AMAS); N-succinimidyl 3-maleimide propionate (BMPS);
[N-ε-maleimide caproyloxy]succinimide ester (EMCS); N-[γ-maleimide butyryloxy]succinimide ester (GMBS); succinimidyl-4-[N-maleimide methyl]cyclohexane-1-carboxy-[6-amidocaproate] (LC-SMCC); succinimidyl 6-(3-[2-pyridyldithio]-propionamide)hexanoate (LC-SPDP); m-maleimidebenzoyl-N-hydroxysuccinimide ester (MBS); N-succinimidyl[4-iodoacetyl] aminobenzoate (SIAB); succinimidyl 4-[N-maleimide methyl]cyclohexane-1-carboxylate (SMCC); N-succinimidyl 3-[2-pyridyldithio]-propionamide (SPDP); [N-ε-maleimide caproyloxy]sulfosuccinimide ester (sulfo-EMCS); N-[γ-maleimide butyryloxy]sulfosuccinimide ester (sulfo-GMBS); 4-sulfosuccinimidyl-6-methyl-α-(2-pyridyldithio)toluamide]hexanoate) (sulfo-LC-SMPT); sulfosuccinimidyl 6-(3'-[2-pyridyldithio]-propionamide)hexanoate (sulfo-LC-SPDP); m-maleimidebenzoyl-N-hydroxysulfosuccinimide ester(sulfo-MBS); N-sulfosuccinimidyl[4-iodoacetyl] aminobenzoate(sulfo-SIAB); sulfosuccinimidyl 4-[N-maleimide methyl]cyclohexan-1-carboxylate (sulfo-SMCC); sulfosuccinimidyl 4-[p-maleimidephenyl]butyrate (sulfo-SMPB); ethylene glycol-bis (succinic acid N-hydroxysuccinimide ester) (EGS); disuccinimidyl tartrate (DST); 1,4,7,10-tetraazacyclododecan-1,4,7,10-tetraacetic acid (DOTA); diethylenetriamine pentaacetic acid (DTPA); N-maleimide caproyl-valyl-citrullyl-p-aminobenzyl carbamate p-nitrophenyl ester (mc-Val-Cit-PABC-PNP); and thiourea.

Among them, SPDP, BMPS, GMBS or mc-Val-Cit-PABC-PNP are preferred as a group for introducing a thiol-reactive group.

The binding of the amino group and the group for introducing a thiol-reactive group can be carried out, for example, through a condensation reaction, an addition reaction, or the like. More specifically, the binding of the amino group and the group for introducing a thiol-reactive group can be carried out, for example, by adding an excess amount of BMPS to the drug bound to a carbon chain having an amino group, incubating the mixture at room temperature for 2 to 4 hours, and, after the amino group is reacted completely, removing unreacted BMPS by ultrafiltration or the like.

In some embodiments of the present invention, it is possible to introduce the group for introducing a thiol-reactive group to a vesicle as a drug. For example, when the vesicle is a vesicle with a lipid having an amino group at the end, the binding of the amino group and the group for introducing a thiol-reactive group can be carried out through a condensation reaction, an addition reaction, or the like. More specifically, the binding may be carried out, for example, by adding an excess amount of BMPS to the drug, incubating the mixture at room temperature for 2 to 4 hours, and, after the amino group is reacted completely, removing unreacted BMPS by ultrafiltration or the like.

When the antibody or antigen-binding fragment thereof has an -SH group, the binding of the thiol-reactive group and the -SH group can be carried out, for example, through a redox reaction, a condensation reaction, an addition reaction, or the like. More specifically, the binding may be carried out, for example, by mixing almost equal amount of the antibody or antigen-binding fragment thereof and a drug to which the thiol-reactive group is introduced, and incubating the obtained mixture at room temperature for 4 to 16 hours. The reaction of the excess may be removed by chromatography.

The present invention provides, when the drug has a thiol-reactive group, a method for producing a conjugate of an antigen-binding fragment Fab' with a drug, comprising reacting the antigen-binding fragment Fab' with the thiol-reactive group of the drug.

The conjugate of Fab' with a drug can be separated from unreacted substances such as Fab' or the drug using separation techniques such as size exclusion chromatography or HPLC.

### (Medicament for therapy for disease)

The conjugate of the present invention may be used as it is or as a mixture with a pharmaceutically acceptable carrier (e.g., excipient) or the like to prepare a medicament.

The conjugate of the present invention may be used as a medicament such as a therapeutic agent for a disease of muscle.

Examples of the disease of muscle include, but are not particularly limited to, a muscular disease, a muscular atrophy disease, an arteriosclerotic disease, and a cardiac-related disease.

Examples of the muscular disease include muscular dystrophy (e.g., Duchenne muscular dystrophy, Becker muscular dystrophy, Emery-Dreifuss muscular dystrophy, facioscapulohumeral muscular dystrophy, oculopharyngeal muscular dystrophy, distal muscular dystrophy, limb-girdle muscular dystrophy, Fukuyama type congenital muscular dystrophy, myotonic dystrophy), periodic paralysis, diaphragmatic paralysis, diaphragmatic atony, distal myopathy, myotonia syndrome, mitochondrial disease, and muscle wasting disease.

Examples of the muscular atrophy disease include sarcopenia (age-related muscle atrophy), disuse muscle atrophy, cachexia, amyotrophic lateral sclerosis, and spinal muscular atrophy.

Examples of the arteriosclerotic disease include a peripheral arterial occlusive disease.

Examples of the cardiac-related disease include angina pectoris (including effort angina, variant angina), acute coronary syndrome (including unstable angina, acute myocardial infarction, post-myocardial infarction heart failure), heart failure (including HFrEF, HFpEF, acute heart failure, chronic heart failure, decompensated heart failure), cardiomyopathy (including dilated cardiomyopathy, hypertrophic cardiomyopathy, restrictive cardiomyopathy), cor pulmonale, asymptomatic myocardial ischemia, arrhythmia (including conduction disturbances, sinus node dysfunction, ectopic supraventricular rhythm, atrioventricular block, atrial fibrillation, atrial flutter, re-entry supraventricular tachycardia (SVT, PSVT), Wolff-Parkinson-White (WPW) syndrome, legs and bundle block (bundle branch block and fascicular block), ventricular extrasystoles, ventricular tachycardia, ventricular fibrillation, and sudden cardiac death), valvular abnormalities (including aortic insufficiency, aortic valve stenosis, mitral valve prolapse (MVP), mitral regurgitation, mitral valve stenosis, pulmonary valve insufficiency, pulmonary arterial stenosis, tricuspid regurgitation, tricuspid valve stenosis), endocarditis, cardiac tumors, decreased cardiac function after cardiopulmonary bypass surgery, maintenance of cardiac function and prevention of heart accident during non-drug therapies for severe heart failure (e.g., intra-aortic balloon pumping, ventricular assist device, Batista surgery, cell transplantation, gene therapy, heart transplant).

The conjugate of an anti-CD71 antibody or antigen binding fragment thereof with a substance having anti-cancer activity of the present invention may be used as a medicament for use in treating cancer, such as a therapeutic agent for cancer.

Examples of the cancer include CD71-positive cancer, such as blood cancer (leukemia, red leukemia), lymphoma, colon cancer, breast cancer, mesothelioma, liver cancer, kidney cancer, small-cell lung cancer, malignant melanoma, medulloblastoma, neuroblastoma, cervical cancer, ovarian cancer, glioma, and glioblastoma.

The conjugate of the present invention may be used as a medicament used to treat a liver disease.

Examples of the liver disease include hemochromatosis, Wilson's disease, glycogen storage disease, amino acid metabolism disorder, urea cycle metabolism disorder, porphyria, constitutional jaundice, fibrotic polycystic liver disease, nonalcoholic steatohepatitis, hepatitis B, liver fibrosis, liver cirrhosis, hereditary ATTR amyloidosis (familial amyloid polyneuropathy (FAP)), and α-1 antitrypsin deficiency (AATD).

The drug of the conjugate of the present invention may be a drug targeting various genes, mRNA, miRNA, and a protein (for example, for increasing or decreasing the expression level). Such a drug may be a drug that is possible to act on a target gene or the like and suppress the production of a protein or the like to be originally expressed. Examples of the drug include nucleic acids such as siRNA, shRNA, ASO, aptamer, and miRNA which are against a target such as various genes, mRNA, miRNA and a protein, and modified nucleic acids thereof. The drug may also be a drug that is possible to produce a target protein in a cell or tissue. Examples of the drug include nucleic acids such as mRNA encoding a target protein, and modified nucleic acids thereof.

The target of the drug of the conjugate of the present invention is not particularly limited. Examples of the target include the following:

### <Targets regarding Cancer and Immunology>

Clusterin gene, nucleolin gene, AKT1 protein kinase gene, BIRC5 gene, MAGEC1 gene, MAGEC2 gene, CTAG1 gene, TPBG gene, Hsp27 gene, β-Catenin gene, CXCL12 (SDF-1) gene, STAT-3 gene, PKN3 gene, PLK1 gene, mutated KRAS (G12D) gene, Grb-2 gene, Androgen receptor gene, TGFβ gene (TGFβ-1 gene, TGFβ-2 gene, TGFβ-3 gene), STAT-3 gene, VEGF gene, KSP (Eg5) gene, CEBPA gene, Nek2 gene, p53 gene, MUC1 gene, TPBG gene, HIF-1α gene, RPN2 gene, EphA2 gene, RRM1 gene, CDC45 gene, six-1 gene, IGF-1 receptor gene, HoxA1 gene, IGFBP-2 gene, IGFBP-5 gene, EGF receptor gene, Raf-1 gene, mTOR gene, Bcl-2 gene, Casein kinase-2 gene, KRAS gene, c-Myc gene, COX-2 gene, β-3 tubin gene, ITCH gene, VEGF gene, VEGF receptor 2 gene, SIP1 gene, AGT gene, ERV-9 LTR gene, EVI1 gene, TNF-a gene, PAX-2 gene, Srpx2 gene, IRS-1 gene, survivin gene, DUSP6 gene, HPV E6/E7 gene, HSPA9 gene, mitochondrial RNA (non-coding mitochondrial RNA), EWS FLI1 gene, SRC-3 gene, MDR1 gene, NTRK1 gene, NTRK2 gene, MDX3 gene, NR2F6 gene, MYD88 gene, NOTCH1 gene, β-3 integrin gene, c-FLIP gene, MADD gene, HER2 gene, CCAT2 gene, CTCFL gene, HIF-2α gene, BMI-1 gene, NETO-2 gene, CTFR gene, PD-1 gene, PD-L1 gene, PD-L2 (B7-DC(CD273)) gene, CLTA4 gene, HLA gene (HLA-A, HLA-B, HLA-C, HLA-DR, HLA-DP, HLA-DQ, HLA-E, HLA-G), MCH gene, gene, 4-1BB gene, 4-1 BBL gene, CD3 gene (CD3α, CD3β, CD3γ, CD3δ, CD3ε, CD3CD3ζ gene, IL-6 gene, IL-17 gene, IL-23 gene, ICOS gene, CD70 gene, CD27 gene, OX40 gene, OX40L gene, TL1A gene, DR3 gene, GITRL gene, GITR gene, CD30L gene, CD30 gene, TIM1 gene, TIM1L gene, TIM4 gene, SLAM gene, CD48 gene, CD58 gene, CD2 gene, CD155 gene, CD112 gene, CD226 gene, CD80 (B7-1) gene, CD86 (B7-2) gene, B7-H2 gene, LIGHT gene, HVEM gene, CD40 gene, CD40L gene, Galectin9 gene, CD113, Collagen gene, CD160 gene, LAG3 gene, PSA gene, PSMA gene, PSCA gene, STEAP gene, BIRC5 gene, MAGEC1 gene, MAGEC2 gene, CTAG1 gene, TPBG gene, or molecules such as proteins encoded by these genes, or mRNAs encoding proteins encoded by these genes, proteins known as HDGF family (prototype proteins, HDGF; HRP-1, HRP-2, HRP-3, HRP-4 (HRP=HDGF Related Protein)); and LEDGF (especially, HRP-3)), TrkB receptors, genes or mRNAs encoding these receptors, and miRNA such as miR-34, miR-34a, miR-16, miR-155, miR-17, miR-17-92, miR-215, let-7, miR-34, miR-10b, miR-3157, miR-34, miR-7, miR-21, miR-574-5p, miR-221, miR-484, miR-205, miR-210, miR-3189-3p, miR-3151, miR-199, miR-101, miR-96, miR-182.

### <Targets regarding Liver Disease>

HSP47 gene, α1-antitrypsin gene, ALAS-1 gene, DGAT2 gene, Hydroxy acid oxidase gene, TGFβ-2 gene, Transthyretin gene, PCSK9 gene, or molecules such as proteins encoded by these genes, or mRNAs encoding proteins encoded by these genes, miR-103, miR-107.

### <Targets regarding Skeletal Muscle>

Myostatin gene, dystrophin gene, SMN2 gene, DMPK gene, SOD1 gene, PABPN1 gene, cPLA2 gene, AMPA-type Glu receptor 1 gene, AMPA-type Glu receptor 3 gene, FOXO-1 gene, C9orf72 gene, ActRIIB gene, DUX4 gene, NLRP3 gene, or molecules such as proteins encoded by these genes, or mRNAs encoding proteins encoded by these genes, miR-155, preferably, myostatin gene, or molecules such as proteins encoded by these genes, or mRNAs encoding proteins encoded by these genes.

### <Targets regarding Cardiac Muscle>

Myostatin gene, ApoA gene, STIM1 gene, EGF1 gene, VEGF-A gene, phospholamban gene, serca2a gene, sarcolipin gene, or molecules such as proteins encoded by these genes, or mRNAs encoding proteins encoded by these genes, β1-adrenergic receptor, TL receptor 4 or a gene or mRNA encoding these receptors, miR-15, miR-195, miR-208, miR-92a, miR-34a, miR-25, miR-486, preferably, myostatin gene, or molecules such as proteins encoded by these genes, or mRNAs encoding proteins encoded by these genes.

The medicament containing the conjugate of the present invention may contain a pharmacologically acceptable carrier (e.g., an excipient). Examples of the excipient which may be contained in the medicament include a salt, a solvent (e.g., water and ethanol), a buffering agent, sugar and sugar alcohol, a detergent, an isotonic agent, a preservative, an antioxidant, a chelating agent and an excipient. The medicament containing the conjugate of the present invention may be administered, for example, intravenously, subcutaneously, intraperitoneally or intramuscularly. Accordingly, the medicament containing the conjugate of the present invention may be formulated according to routine procedures as a medicament for intravenous administration, subcutaneous administration, intraperitoneal administration or intramuscular administration. According to the present invention, the conjugate may be prepared prior to use as a medicament using a medium suitable for administration (for example, sterile pyrogen-free water). The composition of the present invention may contain an excipient in the same manner as the medicament.

### (Method)

The present invention provides a method for delivering a drug to muscle (for example, cardiac muscle and gastrocnemius muscle) in a subject, comprising administering a conjugate of an anti-CD71 antibody or an antigen-binding fragment thereof with a drug to the subject. In the present invention, the anti-CD71 antibody or the antigen-binding fragment thereof may be, for example, Fab'.

The present invention provides a method for delivering a drug to hepatocytes, immune cells such as B cells or T cells, or cancer cells in a subject, comprising administering a conjugate of an anti-CD71 antibody or an antigen-binding fragment thereof with a drug to the subject. In the present invention, the anti-CD71 antibody or the antigen-binding fragment thereof may be, for example, Fab'.

The daily dose of the conjugate of the present invention varies depending on the condition and bodyweight of the patient, type of the drug, the route of administration and the like. When the conjugate is administered intramuscularly, for example, to a patient for therapeutic purposes of a disease of muscle, the daily dose for an adult (weighing about 60 kg) may be about 1 to about 1000 mg as a drug in the conjugate of the present invention, preferably about 3 to about 300 mg, more preferably about 10 to about 200 mg. The daily dose may be divided to administer into multiple times (e.g., once, twice or three times).

The present invention provides a method for increasing quantity of muscle in the muscle (for example, cardiac muscle and gastrocnemius muscle) of a subject, comprising administering the conjugate of an anti-CD71 antibody or an antigen-binding fragment thereof with siRNA or shRNA against myostatin to the subject. The present invention provides a method for treating a peripheral arterial disease in a subject in need thereof, comprising administering a conjugate of an anti-CD71 antibody or an antigen-binding fragment thereof with siRNA or shRNA against myostatin to the subject. In the present invention, the anti-CD71 antibody or the antigen-binding fragment thereof may be, for example, Fab'.

The present invention provides a method for treating cancer in a subject, comprising administering a conjugate of an anti-CD71 antibody or an antigen-binding fragment thereof with a substance having anti-cancer activity to the subject.

The present invention provides a method for treating a liver disease in a subject, comprising administering a conjugate of an anti-CD71 antibody or an antigen-binding fragment thereof with a substance having a preventive and/or therapeutic effect for the liver disease to the subject.

The present invention provides a method for treating an immune disease in a subject, comprising administering a conjugate of an anti-CD71 antibody or an antigen-binding fragment thereof with a substance having a preventive and/or therapeutic effect for the immune disease to the subject. In a specific aspect, before administering the conjugate with the substance having a preventive and/or therapeutic effect for the immune disease, CD71 may be induced in immune cells.

### (Use)

The present invention provides use of a conjugate of an antibody binding to a cell surface antigen or an antigen-binding fragment thereof with a drug, for delivering the drug into the cell. The present invention provides use of a conjugate of an antibody binding to a cell surface antigen or an antigen-binding fragment thereof with a drug, for delivering the drug into the cell. In this aspect, the cell may be a cell which incorporates a membrane protein into the cell by endocytosis. Further, in this aspect, the cell surface antigen is a membrane protein (for example, a receptor), and the membrane protein may be incorporated into the cell by endocytosis. In the present invention, the antibody binding to a cell surface antigen or an antigen-binding fragment thereof may be, for example, Fab'.

The present invention provides use of an anti-CD71 antibody or an antigen-binding fragment thereof for use in delivering a drug to muscle (for example, cardiac muscle and gastrocnemius muscle). The present invention provides use of an anti-CD71 antibody or an antigen-binding fragment thereof for use in increasing quantity of muscle. The present invention provides use of an anti-CD71 antibody or an antigen-binding fragment thereof in manufacture of a composition for use in delivering a drug to muscle (for example, cardiac muscle and gastrocnemius muscle). The present invention provides use of an anti-CD71 antibody or an antigen-binding fragment thereof in manufacture of a medicament for use in increasing quantity of muscle. The present invention provides use of the conjugate of an anti-CD71 antibody or an antigen-binding fragment thereof with siRNA or shRNA against myostatin for use in treating peripheral arterial disease in a subject in need thereof. The present invention further provides use of an anti-CD71 antibody or an antigen-binding fragment thereof in the manufacture of a medicament for use in treating peripheral arterial disease in a subject in need thereof. The present invention further provides use of the conjugate of an anti-CD71 antibody or an antigen-binding fragment thereof with siRNA or shRNA against myostatin in the manufacture of a medicament for use in treating peripheral arterial disease in a subject in need thereof. According to the present invention, the antigen-binding fragment thereof may be, for example, Fab'.

The present invention provides use of an anti-CD71 antibody or an antigen-binding fragment thereof, for use in delivering a drug to hepatocytes, immune cells such as B cells or T cells, or cancer cells (in particular, CD71 positive cells). The present invention provides use of an anti-CD71 antibody or an antigen-binding fragment thereof, in manufacture of a composition for use in delivering a drug to hepatocytes, immune cells such as B cells or T cells, or cancer cells (in particular, CD71 positive cells). The present invention provides use of an anti-CD71 antibody or an antigen-binding fragment thereof and a drug, or use of the conjugate of an anti-CD71 antibody or an antigen-binding fragment thereof with a drug, in manufacture of a composition for use in delivering the drug to hepatocytes, immune cells such as B cells or T cells, or cancer cells (in particular, CD71 positive cells). The present invention provides use of an anti-CD71 antibody or an antigen-binding fragment thereof and a drug, or use of the conjugate of an anti-CD71 antibody or an antigen-binding fragment thereof with a drug, in manufacture of a medicament for use in treating cancer in a subject in need thereof. In the delivery to cancer cells or therapeutic applications for cancer, the drug may be a substance having anti-cancer activity.

### Examples

Hereinafter, the present invention will be described based on the examples without no intention to limit the invention.

### Example 1: Preparation of anti-CD71 antibody-drug conjugate

In this example, an anti-CD71 antibody-siRNA conjugate was prepared as an example of the anti-CD71 antibody-drug conjugate.

The conjugates prepared in this example as nonlimiting examples were as follows. Any of the following conjugates is a conjugate of an antibody fragment with RNA, specifically a conjugate of Fab' and RNA which are linked via a linker. The conjugates represented by the following formulae (I) to (IV) are a conjugate in which Fab' and RNA are linked via a maleimide linker, a Val-Cit linker, an SS linker and a DMSS linker, respectively.

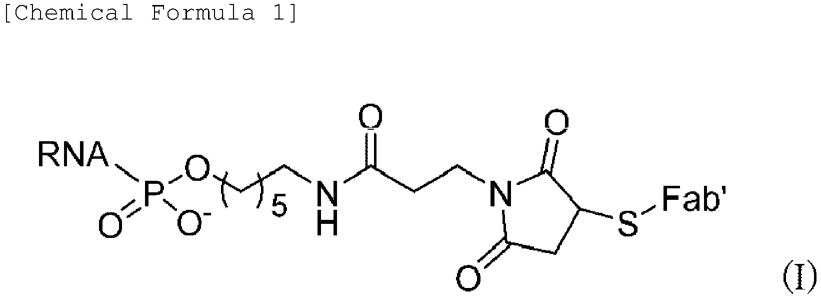

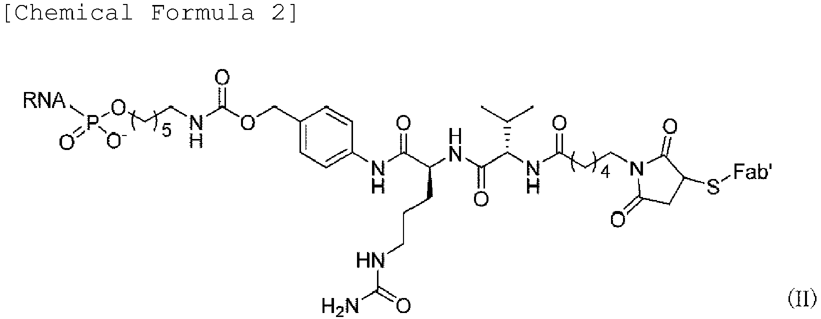

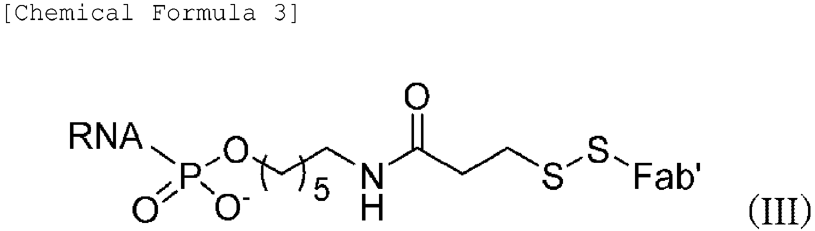

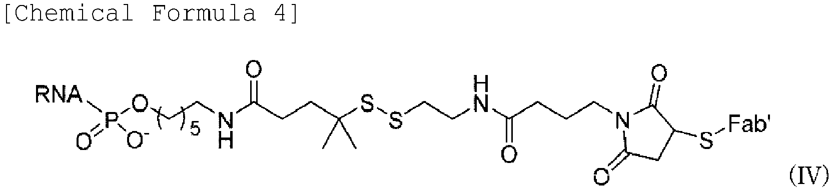

### (1) Anti-CD71 antibody

The anti-CD71 antibody used in this example was anti-CD71 antibody (clone R17 217.1.3) purchased from Bio X Cell (West Lebanon, NH). The control IgG used in this example was isotype control IgG₂ₐ (BP0089) purchased from Bio X Cell. The control IgG is sometimes herein referred to as "IgG₂ₐ", and described as "IgG" in Table 1 below.

### (2) siRNA

The sequences of siRNAs against ApoB, hypoxanthine-phosphoribosyl-transferase (HPRT) and myostatin (MSTN), and the sequence of siRNA (siNC) which was used as a negative control in this example were as follows.

### 1) siApoB

sense strand: 5'-GgAaUcUuAuAuUuGaUcCaA-(CH₂)₆NH₂-3' (SEQ ID NO: 1);
antisense strand: 5'-puUgGaUcAaAuAuAaGaUuCcsCsu-3' (SEQ ID NO: 2);

### 2) siHPRT

sense strand: 5'-UcCuAuGaCuGuAgAuUuUaU-(CH₂)₆NH₂-3' (SEQ ID NO: 3);
antisense strand: 5'-paUaAaAuCuAcAgUcAuAgGasAsu-3' (SEQ ID NO: 4);

### 3) siMSTN

sense strand: 5'-GaGuAuGcUcUaGuAaCgUaU-(CH₂)₆NH₂-3' (SEQ ID NO: 5);
antisense strand: 5'-aUaCgUuAcUaGaGcAuAcUcsAsa-3' (SEQ ID NO: 6);

### 4) siNC

sense strand: 5'-UgUaAuAaCcAuAuCuAcCuU-(CH₂)₆NH₂-3' (SEQ ID NO: 7);
antisense strand: 5'-aAgGuAgAuAuGgUuAuUaCasAsa-3' (SEQ ID NO: 8)

In the sequences represented by SEQ ID Nos. 1 to 8 above, 2'-O-methyl-modified nucleotides are shown in lower case letters, 2'-fluoro-modified nucleotides are underlined, and phosphate and phosphorothioate linking groups are shown in letters p and s, respectively. 2'-O-methyl-modification and 2'-fluoro-modification were introduced to increase stability of siRNA in blood. It should be noted that only nucleotide sequences are shown in the attached Sequence Listing.

### (3) Modification of siRNA for preparation of antibody_ conjugate

To the C6 amino chain at the 3'-side of each sense strand described above, the following compounds were reacted to obtain a sense strand having a thiol-reactive group: N-succinimidyl 3-maleimide propionate (BMPS) (Acme Bioscience, Inc., Palo Alto, CA), N-succinimidyl 3-[2-pyridyldithio]propionate (SPDP) (Thermo Fisher Scientific Inc., Waltham, MA), N-maleimide caproyl-valyl-citrullyl-p-aminobenzyl carbamate p-nitrophenyl ester (mc-Val-Cit-PABC-PNP), or N-succinimidyl 4-methyl-4-(2-pyridyldithio)pentanate (SynChem, Inc., Elk Grove Village, IL). Further, 4-methyl-4-(2-pyridyldithio)pentanate-modified siRNA was reduced with 2-mercaptoethanol, and then reacted with BMPS to form a maleimide group, which was referred to as dimethyl SS (DMSS) linker. However, unless otherwise specified, BMPS was used to form a linker between siRNA and an antibody. All reactions were monitored by high performance liquid chromatography-mass spectrometry (HPLC-MS), and remaining reagents were removed using Amicon Ultra ultrafiltration devices (MWCO 3K, Millipore). The sense and antisense strands of siRNA were annealed. In this way, as shown in Figure 1A, molecules to which various linkers are bound each at 3' end of the sense strand were obtained. These linkers have reactivities to a thiol group.

### (4) Preparation of antibody-siRNA conjugate

The conjugate was prepared according to the scheme shown in Figure 1B. Specifically, clone R17 was subjected to pepsin degradation to obtain F(ab')₂, which was further reduced with cysteamine to obtain Fab' fragments. The obtained Fab' fragments have two thiol groups which can be used for preparation of the conjugate. The Fab' fragments were purified using Sephadex G-25 column equilibrated with a buffer (30 mM HEPES (pH 7.0), 150 mM NaCl), and reacted with the maleimide group or (2-pyridyldithio)pentanate group of the siRNA obtained above to be conjugated in the elution buffer. These reactions were monitored by size exclusion chromatography-HPLC using TSKgel G2000SWxL (7.8 mm × 300 mm, TOSOH) with 20 mM phosphate-300 mM NaCl buffer (pH 7.0). The reactant was incubated overnight at room temperature, and the Fab'-siRNA conjugate was subjected to SEC-HPLC to separate from unreacted Fab' and siRNA (see Figure 1C). The separation of Fab'-siRNA conjugate from unreacted Fab' and siRNA was confirmed (see Figure 1D). The Fab'-siRNA conjugate was concentrated using Amicon Ultra ultrafiltration device (MWCO 10kDa). The siRNA was quantitated using Quant-it RiboGreen RNA assay kit (Life technologies, Carlsbad, CA). The Fab' was quantitated using ATTO-TAG FQ Derivatization Reagent (FQ; 3-(2-furoyl) quinoline-2-carboxaldehyde) (Invitrogen, Carlsbad, CA). Hereinafter, the concentration and weight of conjugate are expressed as the concentration and weight of siRNA. Plasma siRNA was extracted with Trizol, and then reverse transcribed using TaqMan MicroRNA Reverse Transcription Kit (Life technologies, Carlsbad, CA). These samples were quantitated by real-time PCR. According to Figure 1E, it was found that the measured absorption spectrum of Fab' of the anti-CD71 antibody has a peak at 280 nm, and according to Figure 1F, it was found that the measured absorption spectrum of siHRPT has a peak at 260 nm. Further, according to Figure 1G, it was found that the isolated clone R17 Fab'-siRNA conjugate shows the spectrum having both of absorption spectra added together. Some conjugates were prepared using other various linkers in the same manner, and isolation of clone R17 Fab'-siRNA conjugates were verified.

The result of verification of the ratios of molecular numbers of siRNA to protein was as shown in Table 1.

**[Table 1]**

| Molecule | Linker | siRNA/protein |
|---|---|---|
| anti-CD71 Fab'-siApoB | maleimide | 1.4 |
| anti-CD71 Fab'-siHPRT | maleimide | 1.4 |
| anti-CD71 Fab'-siHPRT | Val-Cit | 1.4 |
| anti-CD71 Fab'-siHPRT | SS | 2.2 |
| anti-CD71 Fab'-siHPRT | DMSS | 1.6 |
| anti-CD71 Fab'-siNC | maleimide | 1.0 |
| anti-CD71 Fab'-siMSTN | maleimide | 1.3 |
| IgG Fab'-siHPRT | maleimide | 1.2 |

| | | |
|---|---|---|
| * In the Table, a linker of formula (I) is referred to as a maleimide linker, a linker of formula (II) is referred to as a Val-Cit linker, a linker of formula (III) is referred to as a SS linker, and a linker of formula (IV) is referred to as a DMSS linker. | | |

As shown in Table 1, it has been found that one or two siRNAs were conjugated to two thiol groups present per Fab'.

### Example 2: CD71 binding assay

In this example, the binding ability of clone R17 Fab'-siRNA conjugate prepared above against CD71 was verified. As the conjugate in this example, the clone R17 Fab'-siHPRT linked with the maleimide linker was used.

Using a 96-well black flat-bottom immuno plate (Corning 3925), recombinant mouse CD71 (50 µL of 5 µg/L; Sino Biological Inc., Beijing, China) was incubated overnight at 4°C. The plate was washed twice with Dulbecco's phosphate buffered saline (D-PBS)(-) containing 0.05% Tween 20, and then blocked at room temperature for 1 hour with D-PBS(-) containing 1% bovine serum albumin (BSA). After washing twice with D-PBS(-) containing 0.05% Tween 20, the sample of the conjugate (100 ng in siRNA equivalent) was incubated at room temperature for 2 hours under the presence or absence of competing reagent in the wells. After washing four times with D-PBS(-) containing 0.05% Tween 20, RiboGreen solution was added to the wells for siRNA detection, and fluorescence (excitation at 485 nm, fluorescence at 535 nm) was measured using Envision 2104 Multilabel Reader (PerkinElmer). For detection of binding of the anti-CD71 antibody, 4 µg/mL of Alexa Fluor 488 labeled goat anti-rabbit IgG (Invitrogen, Carlsbad, CA) was added and incubated for 1 hour. Fluorescence was observed after the resultant was washed four times. Non-specific binding was assessed using CD71 uncoated wells. EC₅₀ was calculated using GraphPad Prism 6.

As shown in Figure 2A, EC₅₀ for the binding of clone R17 was 1.5 nM. This suggests that CD71 covering the plate maintains the native conformation. In contrast, when clone R17 Fab'-siHPRT was used as the conjugate, Ribogreen solution diluted 200-fold with PBS was added to each well, and the fluorescence from the Ribogreen which was generated when it bound to the RNA of the conjugate bound to CD71 protein was quantitated in a plate reader, EC₅₀ was 29 nM. In contrast, when IgG₂ₐ-siHPRT was used as the conjugate, binding was not observed. The EC₅₀ value of clone R17 was nearly 20 times compared to that of conjugate, however such a difference of affinity is usually caused due to fragmentation to monovalent Fab' (M. Temponi et al., Cancer Res. 52 (1992) 2497-2503).

When the clone R17 Fab'-siHPRT conjugate competed with the clone R17 which is the source of the clone R17 Fab'-siHPRT conjugate (the parent antibody), the clone R17 Fab'-siHPRT conjugate was replaced by the parent antibody depending on the concentration of the parent antibody. Further it was shown that, in the case that the concentration of the parent antibody was significantly higher than that of the clone R17 Fab'-siHPRT conjugate, the clone R17 Fab'-siHPRT conjugate was almost completely replaced by the parent antibody (Figure 2D). This suggests that the parent antibody and the conjugate bind to the same epitope. Furthermore, when the clone R17 Fab'-siHPRT conjugate competed with transferrin (Tf), it was shown only a weak competition, even with increasing concentrations of Tf (Figure 2E). Substantial amounts of Tf are seemingly present in blood. Thus, the binding of the antibody to CD71 stronger than that of Tf is considered important characteristic in considering the application of the antibody to the living body.

### Example 3: Verification of silencing in vitro

GalNAc-siApoB was synthesized as described in J.K. Nair et al., J. Am. Chem. Soc. 136 (2014) 16958-16961. GalNAc-siApoB is known to bind to an asialoglycoprotein receptor on the surface of hepatocytes and be incorporated into the cells to cause silencing in the cells. In this example, by comparison with this molecule, silencing of clone R17 Fab'-siApoB in hepatocyte, as well as silencing of clone R17 Fab'-siHPRT in B cells and T cells were verified. In any of the conjugates, maleimide was used as the linker.

The measurement of silencing was performed as follows. Total RNA was prepared by Trizol extraction method, and using the prepared RNA as templates, reverse transcription was performed with random primers and Superscript II reverse transcriptase (Invitrogen, Carlsbad, CA) to synthesize DNA. Using the resulting DNA as a template, quantitative PCR was performed. ApoB and HPRT quantitation was performed using Prism 7900 Sequence Detection System (Applied Biosystems, Foster City, CA). During the quantitative PCR, the following primers were used as primers.
Forward primer for ApoB amplification (SEQ ID NO: 9):
   5'-AATGTGGAGTATAATGAAGATGGTCTATTT-3'
Reverse primer for ApoB amplification (SEQ ID NO: 10):
   5'-AGTGCTGGGCTGGTGATGTC-3'
Probe for ApoB detection (SEQ ID NO: 11):
   5'-(FAM)-TTGGGACTGGCAGGGAGAGGCTC-(TAMRA)-3'

As used herein, the term "FAM" represents 6-carboxyfloresein, and the term "TAMRA" represents carboxytetramethylrhodamine.
Forward primer for HPRT amplification (SEQ ID NO: 12):
   5'-CTCCTCAGACCGCTTTTTGC-3'
Reverse primer for HPRT amplification (SEQ ID NO: 13):
   5'-TAACCTGGTTCATCATCGCTAATC-3'
Probe for HPRT detection (SEQ ID NO: 14):
   5'-(FAM)-CCGTCATGCCGACCCGCAGT-(TAMRA)-3'

The internal references ACTB, GAPDH and ribosomal protein largeP0 (Rplp0) were quantitated using ACTB, GAPDH, and Rplp0 control reagents (Applied Biosystems, Forster City, CA) respectively. The quantitative values of ApoB and HPRT were standardized using mRNA amount of any one of these.

The cells used for verification of silencing of clone R17 Fab'-siApoB in this example were primary hepatocytes. Further, the expression level of genes was standardized using the expression level of β-actin. Although the expression level of CD71 in this cell is not high, the clone R17 Fab'-siApoB had EC₅₀ of 11 nM in inhibiting of ApoB gene expression and showed 460 silencing at 900 nM (open circles in Figure 3A). On the other hand, GalNAc-siApoB had EC₅₀ of 0.29 nM, and showed 90% silencing at 900 nM (filled circles in Figure 3A).

It has been known that CD71 is induced in both B cells stimulated with anti-IgM and T cells stimulated with L-PHA. Thus, the silencing of clone R17 Fab'-siHPRT was then verified using B cells and T cells where CD71 is expressed by such stimuli.

B cells and T cells were isolated from the spleen of mouse (BALB/cA Jcl, 8 weeks old, male). Cells were isolated using AutoMACS, and B cells and T cells were isolated using B cell isolation kit mouse (Milteny Biotec, Bergisch Gladbach, Germany) and Pan T cell isolation kit II mouse (Milteny Biotec, Bergisch Gladbach, Germany), respectively. The cells were disseminated at a concentration of 10,000 cells/well in a 96-well plate. The B cells were reacted with various concentrations of clone R17 Fab'-siHPRT under the presence of 10 µg/mL of anti-mouse IgM (SouthernBiotech, Birmingham, AL). The T cells were reacted with various concentrations of clone R17 Fab'-siHPRT under the presence of 5 µg/mL of phytohemagglutinin-L₄ (L-PHA) (Wako Pure Chemical Industries, Ltd., Japan). After 72 hours, the expression of mRNA was quantitated by real-time PCR. The results are shown in Figures 3B and C. As the negative control, Fab' of IgG₂ₐ-siHPRT was used.

As shown in Figure 3B, it has been found that in the B cells activated with anti-IgM, HPRT was silenced by clone R17 Fab'-siHPRT. Further, as shown in Figure 3C, it has been found that in the T cells activated with L-PHA, HPRT was silenced by clone R17 Fab'-siHPRT. In Figures 3B and C, filled circles represent the results obtained by anti-CD71 antibody Fab'-siHPRT, and open circles represent the results obtained by IgG₂ₐ Fab'-siHPRT.

### Example 4: silencing in vivo

In mouse, silencing of endogenous genes by anti-CD71 antibody Fab'-siRNA in vivo was verified. As the conjugate in this example, clone R17 Fab'-siApoB linked with the maleimide linker was used.

The clone R17 Fab'-siApoB was intravenously administered to the mouse at a dose of 10 mg/kg bodyweight. As shown in Figure 4A, clone R17 Fab'-siApoB was detected in blood even 24 hours after the administration.

Next, the silencing in liver was confirmed. From the mouse (Balb/c, 7 weeks old, female) to which clone R17 Fab'-siApoB was administered, each tissue was retrieved and the total RNA was extracted by Trizol extraction method. After extraction, mRNA was confirmed by quantitative PCR as described in Example 3.

As a result, as shown in Figure 4B, silencing of ApoB in liver showed a decrease of 44% even 24 hours after intravenous administration.

Next, silencing in various tissues was examined using siRNA against HPRT gene expressed ubiquitously throughout the body. The clone R17 Fab'-siHPRT conjugate was administered intravenously to mice (n=4) at a dose of 10 mg/kg bodyweight. After 24 hours, HPRT silencing was confirmed in liver, cardiac muscle and gastrocnemius muscle, but not confirmed in spleen (see Figure 4C). More specifically, 22% silencing was observed in cardiac muscle, and 39% silencing was observed in gastrocnemius muscle (see Figure 4C). The same result was also observed in soleus muscle. Further, after 48 hours, HPRT silencing was confirmed in cardiac and gastrocnemius muscles, but not confirmed in liver, spleen, and other tissues (see Figure 4C). Silencing 48 hours after intravenous administration was increased to 37% in cardiac muscle, and to 72% in gastrocnemius muscle. Thus, the transient silencing was observed in liver, while the sustained silencing was confirmed in cardiac muscle and gastrocnemius muscle. Then, about 3 days after the intravenous administration, HPRT silencing in cardiac muscle and gastrocnemius muscle reached maximum values (see Figures 4D and E). On the other hand, any significant effect by IgG₂ₐ Fab'-siHPRT conjugate was not confirmed. Further, no silencing by clone R17 Fab'-siNC (negative control) was confirmed (see Figure 4F). The same result was obtained when standardized using GAPDH as the internal reference (see Figure 4G).

The expression of CD71 in the whole body was confirmed by quantitative PCR. Then it became clear that CD71 was highly expressed in cardiac and gastrocnemius muscles (see Figure 5)

### Example 5: Effect of linker in antibody-drug conjugate (ADC)

In this example, the effect of linker in ADC was examined.

The following linkers were compared: a non-cleavable maleimide linker, a Val-Cit linker which is sensitive against cathepsin B and cleaved in endosome, an SS linker which is cleaved under reducing environment, and a DMSS linker which is more sensitive and cleaved under reducing environment (see Figure 1); and the effects of each linker in ADC were confirmed.

In the case of clone R17 Fab'-siHPRT linked with the Val-Cit linker, when the conjugate was administered intravenously at a dose of 10 mg/kg bodyweight, the HPRT silencing in gastrocnemius muscle was 660, while in the case of clone R17 Fab'-siHPRT linked with the maleimide linker, the HPRT silencing was 650 (Figures 6A and B). Thus, it was shown that whether the linker is cleavable or non-cleavable itself does not affect the gene silencing by ADC (see Figure 1A). Further, in the case of the clone R17 Fab'-siHPRT linked with the SS linker, silencing was 270, and in the case of the clone R17 Fab'-siHPRT linked with a DMSS linker, silencing was 480 (Figure 6A). The results were the same in cardiac muscle when the conjugate was intravenously administered at a dose of 10 mg/kg bodyweight (Figure 6C). In cardiac muscle, it was also shown that whether the linker is cleavable or non-cleavable itself does not affect the gene silencing by ADC (Figures 6C and D).

From the results above, it was revealed that whether Fab' is linked or not after the anti-CD71 antibody Fab'-siHPRT linked with the Val-Cit linker is incorporated into the cell does not affect the silencing of siRNA. Further it was suggested that, since there was no large difference in silencing between the cases of cleavable linker and non-cleavable linker, the Fab' does not inhibit the release of siRNA from CD71 after it was incorporated into the endosome. In other words, it is suggested that Fab' is excellent in balance between the binding affinity with an antigen on the cell surface and the releasability from the endosome, thus it is suitable for delivery of a drug to the cell. Further, it has been known that the muscle has low permeability of substances because endothelial cells in the muscle are paved in the vessel wall and there are no holes. However, the Fab'-drug conjugate achieved the drug delivery into muscle. Thus, it was suggested that the Fab'-drug conjugate is excellent in both the efficiency of passing through vascular endothelium and the efficiency of being incorporated into muscle cells.

Furthermore, HPRT silencing effect by the conjugate of clone R17 Fab'-siHPRT linked with the maleimide linker was confirmed over time in gastrocnemius muscle of the mouse to which the conjugate was administered by a single intravenous administration at a dose of 10 mg/kg bodyweight. As a result, as shown in Figure 6E, HPRT silencing in gastrocnemius muscle was sustained even 4 weeks after the administration. Of course, silencing effect against another gene, myostatin, was not observed (Figure 6E, right), thus it was able to confirm that the silencing sustained for 4 weeks was sequence-specific silencing.

Meanwhile, in the mouse to which the conjugate was administered by a single intravenous administration at a dose of 10 mg/kg bodyweight, HPRT silencing in cardiac muscle was observed but gradually weakened (Figure 6F), although HPRT silencing in gastrocnemius muscle was sustained even 4 weeks after the administration. The expression level of myostatin in the cardiac muscle was not significantly changed (Figure 6F).

Therefore, it was revealed that the anti-CD71 antibody Fab'-siRNA exhibit its effect regardless of the linker. Further, the gene silencing by clone R17 Fab'-siHPRT was sustained over a very long period in the target tissue.

### Example 6: Relation between administration route of anti-CD71 antibody Fab'-siRNA and silencing

In this example, the difference in the silencing by administration route was verified by comparing the silencing of anti-CD71 antibody Fab'-siRNA in intravenous administration, intraperitoneal administration and subcutaneous administration. As the conjugate in this example, clone R17 Fab'-siHPRT linked with the maleimide linker was used.

The clone R17 Fab'-siHPRT at a dose of 10 mg/kg bodyweight was administered intravenously (i.v.), intraperitoneally (i.p.) or subcutaneously (s.c.), and 7 days after the administration, silencing was verified. The results were as shown in Figures 7A and B.

As shown in Figure 7A, by any of i.v. administration, i.p. administration, and s.c. administration, high silencing of HPRT was observed in gastrocnemius muscle. Further, as shown in Figure 7B, by any of i.v. administration, i.p. administration, and s.c. administration, high silencing of HPRT was also observed in cardiac muscle. From this result, it was shown that, by any route of administration, the conjugate reached target tissues by blood circulation.

Then, the effect of clone R17 Fab'-siHPRT was verified by intramuscular administration to gastrocnemius muscle. First, 1 µg of clone R17 Fab'-siHPRT was intramuscularly administered to the right gastrocnemius muscle of mouse. When silencing was confirmed 7 days after the administration, HPRT silencing was observed in the right gastrocnemius muscle (Figure 7C). When using IgG₂ₐ Fab'-siHPRT, siHPRT bound to C6 amino chain, or clone R17 Fab'-siMSTN, HPRT silencing did not occur (Figure 7C). Thus, it was found that this silencing was sequence-dependent and CD71-specific. Further, HPRT silencing was not observed in the left gastrocnemius muscle of the mouse (Figure 7D). The dose in this case was equivalent to a dose of 50 µg/kg bodyweight. Thus, the intramuscular administration reduced the effective dose to a sixtieth compared to the dose of 3 mg/kg bodyweight in the intravenous administration (see, Figure 3B) .

When 630 µg of siHPRT bound to C6 amino chain was administered, it was shown the same effect as 1 µg of the anti-CD71 antibody Fab'-siHPRT was administered. This result means that the effect of silencing was enhanced in 10³ order by conjugating the drug with the anti-CD71 antibody. In the mouse to which 40 µg of the anti-CD71 antibody Fab'-siHPRT was intramuscularly administered, 870 of HPRT silencing was confirmed even 14 days after the administration (Figure 7E, left panel). According to Figure 7E right panel, the expression level of myostatin in this mouse is not significantly changed, thus it is found that the silencing observed in Figure 7E, left panel was sequence-specific silencing. Also in this dose, as shown in Figure 7F left panel, silencing was about 200 in the gastrocnemius muscle of the opposite side.

### Example 7: Effect of anti-CD71 antibody Fab'-siMSTN in peripheral artery disease model

In this example, the effect of anti-CD71 antibody Fab'-siMSTN in the peripheral arterial disease model was examined. As the conjugate in this example, the clone R17 Fab'-siMSTN linked with the maleimide linker was used.

To prepare peripheral arterial disease (PAD) model, mouse purchased from CLEA Japan Inc. (C57BL/6J, 10 weeks old, male) was anesthetized with isoflurane, dissected at the proximal and distal portions of the femoral artery and at the proximal portion of deep femoral artery, and ligated, and then sutured closed. Rear-legs ischemia caused by femoral artery ligation (FAL) damages the muscle. This model has been used as a PAD model to verify effects of various medicaments.

To the mouse, 20 µg/foot of clone R17 Fab'-siMSTN (or PBS as a control) was administered once a week intramuscularly for four weeks.

Then, running performance test was carried out using a treadmill (model LE8706, Panlab, Barcelona, Spain). The mouse left habituated to treadmill before the surgery above, and also subjected to treadmill running exercise once a week after the surgery. In each round of the treadmill running exercise, the initial running speed was 5 cm/sec, and then the speed was raised by 5 cm/sec to 40 cm/sec. After finishing experiments, the total running distance was determined.

First, silencing by clone R17 Fab'-siMSTN was confirmed compared to normal mouse, and it was observed that 7 days after intramuscular administration of clone R17 Fab'-siMSTN to gastrocnemius muscle, mRNA expression level of myostatin decreased in a dose-dependent manner (see Figure 8A, left panel). As shown in Figure 8A, right panel, no effect was observed on the expression level of HPRT gene, thus it was found that this silencing effect was sequence dependent.

In the same manner as described above, a peripheral arterial disease (PAD) mouse model in which both of the femoral arteries were ligated was prepared, and clone R17 Fab'-siMSTN was administered intramuscularly to the model according to the regimen described above.

To the peripheral artery disease (PAD) model, 20 µg/foot of clone R17 Fab'-siMSTN was administered once a week for four weeks, and the therapeutic effect was verified. Femoral artery ligation (FAL) decreased the mRNA expression level of myostatin (see Figure 8C) but did not alter the muscle weight (see Figure 8D) in gastrocnemius muscle treated with PBS. However, in the model to which clone R17 Fab'-siMSTN was administered, 17% increase in quantity of muscle was observed, compared to the control group to which PBS was administered (see, Figure 8D).

Next, histological verification was carried out on muscle. Gastrocnemius muscle was retrieved, and fixed with 10% neutral buffered formalin (Wako Pure Chemical Industries, Ltd., Japan). Sections having 4 µm thick were prepared and subjected to hematoxylin and eosin staining, or immunohistological staining with polyclonal rabbit anti-laminin antibody (Abcam plc.). Sections reacted with anti-laminin antibody were treated with Dako Envision Kit (Dako, Denmark), and developed with DAB substrate kit (Nichirei Bioscience INC. Japan). The average cross-sectional area of muscle fibers and the number of regenerated muscle fibers with nuclei at the center in gastrocnemius muscle was measured using HALOTM image analysis system (Indica Labs, Inc. Corrales, NM). The results were as shown in Figures 8E and F. As shown in Figures 8E and F, the average cross-sectional area of muscle fibers in gastrocnemius muscle was increased in PAD models treated with clone R17 Fab'-siMSTN.

When subjected to a running performance test, the untreated PAD model (PBS-administered group) had a total running distance of 1,986 m, while the mouse treated with anti-CD71 antibody Fab'-siMSTN had total running distance of 2,359 m, thus significant increase was confirmed (Figure 8G). The bodyweight and heart weight at this time were 27.09±0.73 g and 119.1±4.3 mg respectively for the untreated PAD model (PBS-administered group) (n=12), and 27.16±0.93 g and 117.7±5.2 mg respectively for the mouse treated with anti-CD71 antibody Fab'-siMSTN (n=12), thus no significant change was observed.

### Example 8: Evaluation of gene silencing by anti-human CD71 antibody Fab'-siRNA in chronic myeloid leukemia cell strain K562

Silencing by anti-human CD71 antibody Fab'-siRNA was evaluated in K562 cells which are the human CD71-expressing cells. The anti-human CD71 antibody used in this example was OKT9 (manufactured by Bio X Cell: BE0023), and the siRNA used was siRNA against human HPRT gene. In the present example, OKT9 Fab'-siRNA conjugate was prepared in the same method as described in Example 1. The linker used was the maleimide linker of formula (I). As the siRNA for human HPRT, siRNA having the sequences represented by SEQ ID Nos. 3 and 4 were used.

In a 96-well plate, various concentrations of OKT9 Fab'-siHPRT and IgG₂ₐFab'-siHPRT were added, then K562 cells were disseminated thereto at a concentration of 1,000 cells/well. After 72 hours, total RNA was extracted using RNeasy 96 Kit (Qiagen, Hilden, Germany), and cDNA was synthesized using Superscript VILO cDNA Synthesis Kit (Invitrogen, Carlsbad, CA). Quantitative PCR was performed using the obtained cDNA as a template, and silencing was evaluated. During quantitative PCR, the following primers and probes were used.
Forward primer for hHPRT amplification (SEQ ID NO: 17):
   5'-CGTCTTGCTCGAGATGTGATG-3'
Reverse primer for hHPRT amplification (SEQ ID NO: 18):
   5'-CCAGCAGGTCAGCAAAGAATT-3'
Probe for hHPRT detection (SEQ ID NO: 19):
   5'-(FAM)-CCATCACATTGTAGCCCTCTGTGTGCTC-(TAMRA)-3'

The quantitative PCR was performed using ViiA7 Real-Time PCR system (Applied Biosystems, Foster City, CA), and silencing efficiency was calculated by comparative Ct method. In the case, 36B4 gene was used as the internal standard. During quantitative PCR, the following primers and probes were used.
Forward primer for h36B4 amplification (SEQ ID NO: 20):
   5'-CGTCTTGCTCGAGATGTGATG-3'
Reverse primer for h36B4 amplification (SEQ ID NO: 21):
   5'-CCAGCAGGTCAGCAAAGAATT-3'
Probe for h36B4 detection (SEQ ID NO: 22):
   5'-(FAM)-CCATCACATTGTAGCCCTCTGTGTGCTC-(TAMRA)-3'

The results were as shown in Figure 9. As shown in Figure 9, siRNA against hHPRT gene conjugated to a Fab' fragment of OKT9 strongly suppressed the expression of HPRT in K562 cells.

Thus, it is clear that, by using a conjugate of the OKT9 antibody or antigen-binding fragment thereof with a drug, the drug can be effectively delivered to cancer cells.

### Example 9: Evaluation of gene silencing by antisense oligo nucleic acid (ASO)

In mouse, silencing of endogenous gene by the conjugate of anti-CD71 antibody Fab'-ASO in vivo was examined.

As the anti-CD71 antibody in this example, clone R17 was used. As the ASO, the antisense oligo nucleic acid against MALAT1 gene (herein sometimes referred to as "MALAT1-ASO") was used.

Antisense oligo nucleic acid against MALAT1 gene
5'-aCTAgttcactgaaTGC-3' (SEQ ID NO: 23) {wherein, the upper case letters mean that nucleic acids are BNA^{NC}, and the lower case letters means that nucleic acids are DNA; all nucleic acids have phosphorothioate modification; and the underlined adenine, used as a spacer, is linked to a linker and base C with phosphodiester bonds.}

### (1) Preparation of maleimide group-introduced ASO

To the aqueous solution of MALAT1-ASO to which an amino group was introduced by linking the C6 amino chain via spacer a (adenine) (1680 nmol, synthesized on commission by GeneDesign Inc.), DMSO, 10×PBS aqueous solution, and distilled water were added. Then, BMPS (1680 nmol) dissolved in DMSO was added and reacted at room temperature for 30 minutes. Ultrafiltered dialysis was performed to remove the residue and the like of the reagent to obtain a maleimide group-introduced MALAT1-ASO (1630 nmol) of interest.

### (2) Preparation of anti-CD71 antibody Fab'

The F(ab) '₂ of clone R17 (78.2 mg) was reduced with 2-aminoethanethiol, and Fab' of interest was fractionated by size exclusion column chromatography. The obtained Fab' was used as it is for the conjugate reaction with MALAT1-ASO.

### (3) Preparation of anti-CD71 antibody Fab'-ASO

To the prepared clone R17 Fab', the maleimide group-introduced MALAT1-ASO (1470 nmol) was added and reacted at room temperature overnight. The reaction system was concentrated and separated the product of interest by size exclusion column chromatography. The fraction of interest was mixed and concentrated, and then the solvent was substituted with PBS to obtain a conjugate of anti-CD71 antibody Fab'-ASO (250 nmol) of interest (see formula (I') below). 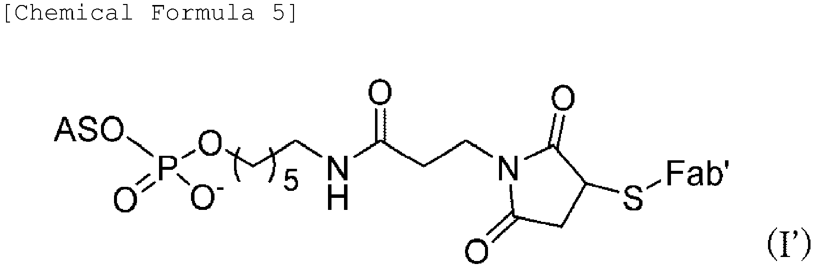 {wherein, "(ASO)" represents antisense oligo (ASO); and ASO is linked to a linker at the 5' end.}

The clone R17 Fab'-MALAT1-ASO was administered intravenously to the mice (Balb/c, 7 weeks old, female) (n=4) at a dose of 10 mg/kg bodyweight. Next, the silencing in heart and gastrocnemius muscle was confirmed. From the mouse to which clone R17 Fab'-MALAT1-ASO was administered, each tissue was rettieved and the total RNA was extracted by Trizol extraction method. After extraction, mRNA was confirmed by quantitative PCR as described in Example 3. β-actin was used as the internal standard.

As a result, as shown in Figure 10, the expressions of Malat1 in heart and gastrocnemius muscle were silenced 89% and 65% respectively, even 48 hours after intravenous administration.

The present disclosure includes, *inter alia,* the following items:
[Item 1]
   A conjugate of an anti-CD71 antibody or an antigen-binding fragment thereof with a drug.
[Item 2]
   The conjugate according to item 1, wherein the antigen-binding fragment is Fab'.
[Item 3]
   The conjugate according to item 1 or 2, wherein the anti-CD71 antibody or the antigen-binding fragment thereof is Fab' of the anti-CD71 antibody.
[Item 4]
   The conjugate according to any one of items 1 to 3, wherein the drug is a nucleic acid.
[Item 5]
   The conjugate according to any one of items 1 to 4, wherein the drug is RNA.
[Item 6]
   The conjugate according to any one of items 1 to 5, wherein the drug is mRNA.
[Item 7]
   The conjugate according to any one of items 1 to 5, wherein the drug is siRNA.
[Item 8]
   The conjugate according to any one of items 1 to 4, wherein the drug is an antisense oligonucleotide.
[Item 9]
   The conjugate according to any one of items 1 to 8, wherein the anti-CD71 antibody or the antigen-binding fragment thereof is linked to the drug via a linker.
[Item 10]
   A composition comprising the conjugate according to any one of items 1 to 9, for use in delivering a drug to at least one selected from cardiac muscle and skeletal muscle.
[Item 11]
   Use of an anti-CD71 antibody or an antigen-binding fragment thereof, for delivering a drug to at least one selected from cardiac muscle and skeletal muscle.
[Item 12]
   An anti-CD71 antibody or antigen-binding fragment thereof, for use in delivering a drug to at least one selected from cardiac muscle and skeletal muscle.
[Item 13]
   A medicament comprising the conjugate according to any one of items 1 to 9.
[Item 14]
   The medicament according to item 13, wherein the medicament is a preventive and/or therapeutic agent for a disease of muscle.
[Item 15]
   A method for prevention and/or therapy for a disease of muscle, comprising administering an effective amount of the medicament according to item 13 to a mammal.
[Item 16]
   Use of the conjugate according to any one of items 1 to 9 for manufacturing a preventive and/or therapeutic agent for a disease of muscle.
[Item 17]
   The conjugate according to any one of items 1 to 9 for use in prevention and/or therapy for a disease of muscle.
[Item 18]
   A method for producing a conjugate of Fab' of an anti-CD71 antibody with a drug, comprising reacting a thiol group of the Fab' with a drug having a functional group reactive with the thiol group, or reacting a thiol group of the Fab' with a drug bound to a linker having a functional group reactive with the thiol group.

## Claims

1. A conjugate of an anti-CD71 antibody or an antigen-binding fragment thereof with a drug formed by a non-cleavable linker which has a thiol-reactive group that is a maleimide group, wherein the thiol-reactive group means a functional group having reactivity with a thiol group of the anti-CD71 antibody or the binding fragment thereof, and wherein the drug is a nucleic acid.

2. The conjugate according to claim 1, wherein the anti-CD71 antibody or the antigen-binding fragment thereof is Fab' of the anti-CD71 antibody.

3. The conjugate according to any one of claims 1 to 2, wherein the anti-CD71 antibody or the antigen-binding fragment thereof binds to CD71 at a different site from the site transferrin binds to.

4. The conjugate according to any one of claims 1 to 3, wherein the drug is RNA.

5. The conjugate according to any one of claims 1 to 4, wherein the drug is mRNA.

6. The conjugate according to any one of claims 1 to 4, wherein the drug is siRNA.

7. The conjugate according to any one of claims 1 to 3, wherein the drug is an antisense oligonucleotide.

8. A medicament comprising the conjugate according to any one of claims 1 to 7.

9. The medicament according to claim 8, wherein the medicament is a preventive and/or therapeutic agent for a muscular disease, a muscular atrophy disease, an arteriosclerotic disease, or a cardiac-related disease.

10. The conjugate according to any one of claims 1 to 7 for use in prevention and/or therapy for a muscular disease, a muscular atrophy disease, an arteriosclerotic disease, or a cardiac-related disease.

11. The conjugate for use according to claim 10, wherein the conjugate is delivered to cardiac muscle or skeletal muscle.

12. A method for producing a conjugate of an anti-CD71 antibody or an antigen-binding fragment thereof with a drug, comprising reacting a thiol group of the antibody or the antigen-binding fragment thereof with a drug bound to a linker having a functional group reactive with the thiol group (thiol-reactive group),
wherein the thiol-reactive group is a maleimide group, and wherein the drug is a nucleic acid.


SEQUENCE LISTING

<110>  GenAhead Bio, Inc.

<120>  Antibody-drug conjugate

<130>  PG25-9001WO

<150>  JP2016-122187
<151>  2016-06-20

<160>  31

<170>  PatentIn version 3.5

<210>  1
<211>  21
<212>  RNA
<213>  Artificial Sequence

<220>
<223>  siApoB sense



<220>
<221>  modified_base
<222>  (1)..(21)
<223>  2'-fluoro modifications at positions 1, 3, 5, 7, 9, 11, 13, 15,
       17, 19, 21

<220>
<221>  modified_base
<222>  (1)..(21)
<223>  2'-O-methyl modifications at positions 2, 4, 6, 8, 10, 12, 14,
       16, 18, 20

<400>  1
ggaaucuuau auuugaucca a                                                21


<210>  2
<211>  23
<212>  RNA
<213>  Artificial Sequence

<220>
<223>  siApoB antisense



<220>
<221>  modified_base
<222>  (1)..(23)
<223>  2'-fluoro modifications at positions 2, 4, 6, 8, 10, 12, 14,
       16, 18, 20, 22

<220>

<221>  modified_base
<222>  (1)..(23)
<223>  2'-O-methyl modifications at positions 1, 3, 5, 7, 9, 11, 13, 15,
       17, 19, 21, 23

<400>  2
uuggaucaaa uauaagauuc ccu                                              23


<210>  3
<211>  21
<212>  RNA
<213>  Artificial Sequence

<220>
<223>  siHPRT sense


<220>
<221>  modified_base
<222>  (1)..(21)
<223>  2'-fluoro modifications at positions 1, 3, 5, 7, 9, 11, 13, 15,
       17, 19, 21

<220>
<221>  modified_base
<222>  (1)..(21)
<223>  2'-O-methyl modifications at positions 2, 4, 6, 8, 10, 12, 14,
       16, 18, 20

<400>  3
uccuaugacu guagauuuua u                                                21


<210>  4
<211>  23
<212>  RNA
<213>  Artificial Sequence

<220>
<223>  siHPRT antisense


<220>
<221>  modified_base
<222>  (1)..(23)
<223>  2'-fluoro modifications at positions 2, 4, 6, 8, 10, 12, 14,
       16, 18, 20, 22

<220>
<221>  modified_base
<222>  (1)..(23)
<223>  2'-O-methyl modifications at positions 1, 3, 5, 7, 9, 11, 13,
       15, 17, 19, 21, 23

<400>  4
   auaaaaucua cagucauagg aau                                              23


<210>  5
<211>  21
<212>  RNA
<213>  Artificial Sequence

<220>
<223>  siMSTN sense



<220>
<221>  modified_base
<222>  (1)..(21)
<223>  2'-fluoro modifications at positions 1, 3, 5, 7, 9, 11, 13, 15,
       17, 19, 21

<220>
<221>  modified_base
<222>  (1)..(21)
<223>  2'-O-methyl modifications at positions 2, 4, 6, 8, 10, 12, 14,
       16, 18, 20

<400>  5
gaguaugcuc uaguaacgua u                                                21


<210>  6
<211>  23
<212>  RNA
<213>  Artificial Sequence

<220>
<223>  siNSTN antisense



<220>
<221>  modified_base
<222>  (1)..(23)
<223>  2'-fluoro modifications at positions 2, 4, 6, 8, 10, 12, 14,
       16, 18, 20, 22

<220>
<221>  modified_base
<222>  (1)..(23)
<223>  2'-O-methyl modifications at positions 1, 3, 5, 7, 9, 11, 13,
       15, 17, 19, 21, 23

<400>  6
auacguuacu agagcauacu caa                                              23


<210>  7
<211>  21
<212>  RNA

<213>  Artificial Sequence

<220>
<223>  siNC sense



<220>
<221>  modified_base
<222>  (1)..(21)
<223>  2'-fluoro modifications at positions 1, 3, 5, 7, 9, 11, 13, 15,
       17, 19, 21

<220>
<221>  modified_base
<222>  (1)..(21)
<223>  2'-O-methyl modifications at positions 2, 4, 6, 8, 10, 12, 14,
       16, 18, 20

<400>  7
uguaauaacc auaucuaccu u                                                21


<210>  8
<211>  23
<212>  RNA
<213>  Artificial Sequence

<220>
<223>  siNC antisense



<220>
<221>  modified_base
<222>  (1)..(23)
<223>  2'-fluoro modifications at positions 2, 4, 6, 8, 10, 12, 14,
       16, 18, 20, 22

<220>
<221>  modified_base
<222>  (1)..(23)
<223>  '-O-methyl modifications at positions 1, 3, 5, 7, 9, 11, 13, 15,
       17, 19, 21, 23

<400>  8
aagguagaua ugguuauuac aaa                                              23


<210>  9
<211>  30
<212>  DNA
<213>  Artificial Sequence

<220>
<223>  Forward primer for ApoB amplification

<400>  9

aatgtggagt ataatgaaga tggtctattt                                        30


<210>  10
<211>  20
<212>  DNA
<213>  Artificial Sequence

<220>
<223>  Reverse primer for ApoB amplification

<400>  10
agtgctgggc tggtgatgtc                                                  20


<210>  11
<211>  23
<212>  DNA
<213>  Artificial Sequence

<220>
<223>  Probe for ApoB detection

<400>  11
ttgggactgg cagggagagg ctc                                              23


<210>  12
<211>  20
<212>  DNA
<213>  Artificial Sequence

<220>
<223>  Forward primer for HPRT amplification

<400>  12
ctcctcagac cgcttttttgc                                                  20


<210>  13
<211>  24
<212>  DNA
<213>  Artificial Sequence

<220>
<223>  Reverse primer for HPRT amplification

<400>  13
taacctggtt catcatcgct aatc                                             24


<210>  14
<211>  20
<212>  DNA
<213>  Artificial Sequence

<220>
<223>  Probe for HPRT detection

<400>  14
ccgtcatgcc gacccgcagt                                                   20


<210>  15
<211>  21
<212>  RNA
<213>  Artificial Sequence

<220>
<223>  siMSTN sense

<400>  15
gaguaugcuc uaguaacgua u                                                 21


<210>  16
<211>  23
<212>  RNA
<213>  Artificial Sequence

<220>
<223>  siMSTN antisense

<400>  16
auacguuacu agagcauacu caa                                               23


<210>  17
<211>  21
<212>  DNA
<213>  Artificial Sequence

<220>
<223>  Forward primer for amplification of hHPRT

<400>  17
cgtcttgctc gagatgtgat g                                                 21


<210>  18
<211>  21
<212>  DNA
<213>  Artificial Sequence

<220>
<223>  Reverse primer for amplification of hHPRT

<400>  18
ccagcaggtc agcaaagaat t                                                 21


<210>  19

<211>  28
<212>  DNA
<213>  Artificial Sequence

<220>
<223>  Probe for detecting hHPRT

<400>  19
ccatcacatt gtagccctct gtgtgctc                                          28


<210>  20
<211>  21
<212>  DNA
<213>  Artificial Sequence

<220>
<223>  Forward primer for amplification of h36B4

<400>  20
cgtcttgctc gagatgtgat g                                                 21


<210>  21
<211>  21
<212>  DNA
<213>  Artificial Sequence

<220>
<223>  Reverse primer for amplification of h36B4

<400>  21
ccagcaggtc agcaaagaat t                                                 21


<210>  22
<211>  28
<212>  DNA
<213>  Artificial Sequence

<220>
<223>  Probe for detecting h36B4

<400>  22
ccatcacatt gtagccctct gtgtgctc                                          28


<210>  23
<211>  17
<212>  DNA
<213>  Artificial Sequence

<220>
<223>  ASO for silencing MALAT1

<220>
<221>  modified_base
<222>  (1)..(17)
<223>  BNA(NC) at positions 2 to 4 and 15 to 17

<220>
<221>  modified_base
<222>  (2)..(17)
<223>  Two adjacent bases are linked with phosphorothioate bond

<400>  23
actagttcac tgaatgc                                                    17


<210>  24
<211>  119
<212>  PRT
<213>  Artificial Sequence

<220>
<223>  Heavy chain variable region of OKT9

<400>  24

Glu Val Gln Leu Gln Gln Ser Gly Pro Glu Leu Val Lys Pro Gly Thr
1               5                   10                  15

Ser Met Asn Ile Ser Cys Lys Ala Ser Gly Tyr Thr Phe Thr Asn Tyr
            20                  25                  30

Tyr Met His Trp Val Arg Gln Arg Pro Gly Gln Gly Leu Glu Trp Ile
        35                  40                  45

Gly Trp Ile Tyr Pro Gly Asp Gly Asn Thr His Tyr Asn Glu Lys Phe
    50                  55                  60

Lys Gly Lys Thr Thr Leu Thr Ala Asp Thr Ser Ser Ile Thr Ala Tyr
65                  70                  75                  80

Met Leu Leu Ser Ser Leu Thr Ser Glu Asp Ser Ala Val Tyr Phe Cys
                85                  90                  95

Ala Arg Asp Asn Ser Gly Tyr Val Gly Phe Pro Tyr Trp Gly Gln Gly
            100                 105                 110

Thr Thr Leu Thr Val Ser Ser
        115

<210>  25
<211>  114
<212>  PRT
<213>  Artificial Sequence

<220>
<223>  Light chain variable region of OKT9

<400>  25

Met Asp Ile Lys Met Thr Gln Ser Pro Ser Ser Leu Thr Val Thr Ala
1               5                   10                  15

Gly Glu Arg Val Thr Met Ser Cys Lys Ser Ser Gln Ser Leu Phe Asn
            20                  25                  30

Ser Ala Asn Gln Glu Asn Tyr Leu Thr Trp Tyr Gln Gln Arg Pro Gly
        35                  40                  45

Gln Pro Pro Lys Leu Leu Ile Tyr Trp Ala Ser Thr Arg Asp Ser Gly
    50                  55                  60

Val Pro Asp Arg Phe Thr Gly Ser Gly Ser Gly Thr Asp Phe Pro Leu
65                  70                  75                  80

Thr Ile Ser Ser Val Gln Ala Glu Asp Leu Ala Val Tyr Tyr Cys Gln
                85                  90                  95

Asn Asp Tyr Ser Tyr Pro Leu Thr Phe Gly Ala Gly Thr Lys Leu Glu
            100                 105                 110

Ile Lys

<210>  26
<211>  10
<212>  PRT
<213>  Artificial Sequence

<220>
<223>  HCDR1 of OKT9

<400>  26

Gly Tyr Thr Phe Thr Asn Tyr Tyr Met His
1               5                   10

<210>  27
<211>  17
<212>  PRT
<213>  Artificial Sequence

<220>
<223>  HCDR2 of OKT9

<400>  27

Trp Ile Tyr Pro Gly Asp Gly Asn Thr His Tyr Asn Glu Lys Phe Lys
1               5                   10                  15

Gly

<210>  28
<211>  10
<212>  PRT
<213>  Artificial Sequence

<220>
<223>  HCDR3 of OKT9

<400>  28

Asp Asn Ser Gly Tyr Val Gly Phe Pro Tyr
1               5                   10

<210>  29
<211>  17
<212>  PRT
<213>  Artificial Sequence

<220>
<223>  LCDR1 of OKT9

<400>  29

Lys Ser Ser Gln Ser Leu Phe Asn Ser Ala Asn Gln Glu Asn Tyr Leu
1               5                   10                  15

Thr

<210>  30
<211>  7
<212>  PRT
<213>  Artificial Sequence

<220>

<223>  LCDR2 of OKT9

<400>  30

Trp Ala Ser Thr Arg Asp Ser
1               5



<210>  31
<211>  9
<212>  PRT
<213>  Artificial Sequence

<220>
<223>  LCDR3 of OKT9

<400>  31

Gln Asn Asp Tyr Ser Tyr Pro Leu Thr
1               5